# EUROPEAN PATENT APPLICATION

(11) **EP 4 541 822 A2**
(43) Date of publication of application: **23.04.2025**
(21) Application number: 23822595.7
(22) Date of filing: 16.06.2023
(51) Int. Cl.: C07K 19/00, C07K 16/46, G01N 33/68, A61K 38/16, A61K 47/68, A61P 29/00, A61P 31/04, A61P 31/12, A61P 35/00, A61P 37/02

(54) **PHASE CHANGE ADJUSTING KIT AND USE THEREOF**

(30) Priority: 16.06.2022 CN 202210687061
(71) Applicant: Tsinghua University, Beijing 100084 (CN)
(72) Inventor: LI, Pilong, Beijing 100084 (CN); DU, Juanjuan, Beijing 100084 (CN); LI, Ru, Beijing 100084 (CN)
(74) Representative: Hoffmann Eitle
(86) International application number: PCT/CN2023/000076
(87) International publication number: WO 2023/241023

(57) **Abstract**

Provided in the present invention is a novel phase transition adjusting kit, comprising at least two independent components which can interact with each other to specifically drive themselves and cell surface molecules combined thereon to generate phase separation, so as to effectively enhance the cell surface molecular oligomerization and the regulation and control effect on downstream signals, and achieve higher targeting specificity. Further provided are a method for screening the phase transition adjusting kit, a composition or a test kit comprising said kit, and a disease treatment method or a disease prevention method using said kit.

## Description

### Technical Field

The present invention relates to a novel phase transition adjusting kit, comprising at least two independent components which can interact with each other to specifically drive the kit per se and the cell surface molecules combined thereon to generate phase separation, so as to effectively enhance the oligomerization of the cell surface molecules and the regulation on downstream signals, and to achieve higher targeting specificity. The present invention also relates to a method for screening the phase transition adjusting kit, a composition or a test kit comprising said kit, and a disease treatment method or a disease prevention method using said kit.

### Background Art

Cell surface molecules are common candidate drug targets, but most of them are ultimately failed in developing useful medicines. For example, death receptor 5 (DR5), a member of the tumor necrosis factor (TNF) receptor superfamily, has not yet been drugged. Although several chimeric antibodies or fully human antibodies have been reported for this receptor, such as Drozitumab, Conatumumab, LBY-135, and the recombinant natural ligand Dulanermin, their development were all terminated ultimately because of displaying extremely limited efficacy or even no efficacy for unknown reasons (see, for example, US2007/0031414, EP1922337A). In addition, a tetrameric nanobody TAS266 has also been reported (see, for example, WO2011/098520). This antibody can effectively activate death receptor 5, but cannot specifically target tumors and has high systemic toxicity.

Studies have found that multivalent biological macromolecules can aggregate through internal multivalent domains, via intermolecular or intramolecular interactions, thereby separating from the common solution phase surrounding the molecules to form an independent liquid phase enriched with the macromolecules. This process is called "liquid-liquid phase separation" (LLPS, also referred to as "phase separation" herein) or "phase transition". A large number of small droplets enriched with the macromolecules exist in the liquid phase formed by phase separation, and the diameter of the droplets can reach several microns or even larger. Such highly recognizable small droplets are called "phase transition droplets". To date, there has been no report on the use of biomolecules or the multivalent domains thereof with phase transition adjusting function for enhancing the binding activity of ligands to cell surface molecules, thereby improving useful properties (such as the druggability) of cell surface molecules.

### Summary of the Invention

Through in-depth research, the inventors have found that the use of a phase transition adjusting kit comprising at least two of the following components can not only improve the selectivity and specificity of each component for the candidate target, but also allow the kit to exhibit excellent effects in target selectivity and specificity, cell surface molecule aggregation effect and/or signal pathway manipulation activity, compared with the ligand molecule binding to the same target but having no phase separation adjusting activity.

Therefore, in one aspect, the present invention provides a phase transition adjusting kit, characterized in that the kit comprises multiple components including a first component and a second component, wherein the first component comprises a first multivalent domain and a first ligand that specifically binds to a first cell surface molecule, and the second component comprises a second multivalent domain and a second ligand that specifically binds to a second cell surface molecule, the first cell surface molecule is the same as or different from the second cell surface molecule, and, each of the components comprises no more than one ligand. The kit of the present invention regulates phase transition of the kit itself or the cell surface molecules bound to the kit through interactions between the multivalent domains of the multiple components contained therein.

In some embodiments, the phase transition adjusting kit of the present invention comprises three or more components, wherein the components other than the first component and the second component comprise a domain capable of regulating phase transition via the interaction with the first and/or second multivalent domains, and optionally contain or do not contain a third ligand that specifically binds to a third cell surface molecule.

In some embodiments, the ligands contained in the components in the phase transition adjusting kit of the present invention are covalently linked to the multivalent domains, optionally via a linker, which the linker may be a peptide linker or a non-peptide linker.

In another aspect, after the phase transition adjusting kit of the present invention binds to one or more cell surface molecules targeted by the ligand, the degree of oligomerization of the one or more cell surface molecules is significantly increased as compared with the degree of oligomerization in case of not being bound to the phase transition adjusting kit of the present invention, or in case of binding to the ligand alone which contained in the phase transition adjusting kit of the present invention.

In a preferred embodiment, in the phase transition adjusting kit of the present invention, the multivalent domain in the component comprises at least 2 tandemly linked motifs, for example, the number of the tandemly linked motifs is 3, 4, 5, 6, 7, 8, 9 or 10, and the motifs may be the same with or different from each other.

In a more preferred embodiment, in the phase transition adjusting kit of the present invention, the multivalent domain contained in each component is composed of at least two or more tandemly linked SUMO3 motifs, SIM motifs, PRMH motifs or SH3 motifs. The SUMO3 motif comprises the amino acid sequence shown in SEQ ID NO: 49, or comprises an amino acid sequence having at least 80%, 90%, 95%, or 99% identity to the sequence shown in SEQ ID NO: 49; the SIM motif comprises the amino acid sequence set forth in SEQ ID NO: 50, or comprises an amino acid sequence having at least 80%, 90%, 95%, or 99% identity to the sequence shown in SEQ ID NO: 50; the PRMH motif comprises the amino acid sequence shown in SEQ ID NO: 51, or comprises an amino acid sequence having at least 80%, 90%, 95%, or 99% identity to the sequence set forth in SEQ ID NO: 51; the SH3 motif comprises the amino acid sequence shown in SEQ ID NO: 52, or comprises an amino acid sequence having at least 80%, 90%, 95%, or 99% identity to the sequence shown in SEQ ID NO: 52.

In a preferred embodiment, the multivalent domain comprises or is consisted of an amino acid sequence shown in SEQ ID NO: 2, 4, 22, 24, 55 or 56, or comprises or is consisted of an amino acid sequence having at least 80%, 90%, 95%, 99% identity to the amino acid sequence shown in SEQ ID NO: 2, 4, 22, 24, 55 or 56.

In another preferred embodiment, the first ligand contained in the phase transition adjusting kit of the present invention can specifically bind to tumor necrosis factor receptor, preferably death receptor 5 (DR5) or Fas receptor, more preferably death receptor 5 (DR5).

In another preferred embodiment, the second ligand contained in the phase transition adjusting kit of the present invention is capable of specifically binding to a tumor-associated antigen. In a more preferred aspect, the tumor-associated antigen is selected from the group consisting of CXC motif chemokine receptor 4 (CXCR4), hepatocyte growth factor receptor (c-Met or HGFR), epidermal growth factor receptor (EGFR), epidermal growth factor receptor 2 (HER2), prostate-specific membrane antigen (PSMA), fibroblast activation protein (FAP), carcinoembryonic antigen (CEA), folate receptor alpha (FolR1), melanoma-associated chondroitin sulfate proteoglycan (MCSP), p95HER2, EpCAM, HER3, CD30 or TPBG (5T4), CD19, CD79b, CD20, CD22, CD37, CD38, BCMA and GPRC5D.

In another preferred embodiment, the ligand in the phase transition adjusting kit of the present invention can be a peptide ligand or a non-peptide ligand, wherein the peptide ligand can be selected from one or more of the group consisting of the following: antibodies or antigen-binding fragments thereof, cytokines, growth factors, adhesion molecules, peptide hormones, or polypeptides randomly selected by phage display or yeast display that specifically bind to cell surface molecules; the non-peptide ligand can be selected from one or more of the group consisting of small molecule agonists or antagonists, antisense oligonucleotides or small interfering RNA (siRNA). In a more preferred aspect, the antibody may be selected from monoclonal, polyclonal, human or humanized antibodies, and the antigen-binding fragment may be selected from F(ab')₂, Fab, single-chain variable fragment (scFv), single-domain antibody fragment (VHH or nanobody).

In other preferred embodiments, one or more ligands in the phase transition adjusting kit of the present invention are single chain variable fragments. In other more preferred embodiments, one ligand contained in the phase transition adjusting kit of the present invention comprises the amino acid sequence shown in SEQ ID NO: 53 or an amino acid sequence having at least 80%, 90%, 95%, 99% identity with the amino acid sequence shown in SEQ ID NO: 53, and/or the other ligand comprises the amino acid sequence shown in SEQ ID NO: 54 or an amino acid sequence having at least 80%, 90%, 95%, 99% identity with the amino acid sequence shown in SEQ ID NO: 54. In other more preferred embodiments, one ligand contained in the phase transition adjusting kit of the present invention is consisted of the amino acid sequence shown in SEQ ID NO: 53 or an amino acid sequence having at least 80%, 90%, 95%, 99% identity with the amino acid sequence shown in SEQ ID NO: 53, and/or the other ligand is consisted of the amino acid sequence shown in SEQ ID NO: 54 or an amino acid sequence having at least 80%, 90%, 95%, 99% identity with the amino acid sequence shown in SEQ ID NO: 54.

In a further preferred embodiment, the phase transition adjusting kit of the present invention comprises the following first component and second component, or is consisted of the following first component or second component, wherein the first component comprises the amino acid sequence of positions 27 to 673 of SEQ ID NO: 12, or the amino acid sequence of positions 19 to 457 of SEQ ID NO: 20, or the amino acid sequence of positions 19 to 550 of SEQ ID NO: 28, or comprises an amino acid sequence having at least 80%, 90%, 95%, 99% identity to a sequence selected from positions 27 to 673 of SEQ ID NO: 12, positions 19 to 457 of SEQ ID NO: 20, or positions 19 to 550 of SEQ ID NO: 28; and the second component comprises the amino acid sequence of positions 21 to 373 of SEQ ID NO: 10, or the amino acid sequence of positions 19 to 257 of SEQ ID NO: 18, or the amino acid sequence of positions 27 to 296 of SEQ ID NO: 26, or comprises an amino acid sequence having at least 80%, 90%, 95%, 99% identity to a sequence selected from positions 21 to 373 of SEQ ID NO: 10, positions 19 to 257 of SEQ ID NO: 18, or positions 27 to 296 of SEQ ID NO: 26.

In a preferred embodiment, the ligand in the phase transition adjusting kit of the present invention is covalently linked to the multivalent domain via a linker, and the linker can be selected from a peptide linker or a non-peptide linker.

In a preferred embodiment, the phase transition adjusting kit of the present invention may further comprise a tag that does not affect the functions of the ligand and the multivalent domain and/or a polypeptide that cleaves the tag. In some embodiments, the tag is used to isolate, purify, or identify the multivalent domain. In some embodiments, the tag may be selected from a polyhistidine (Hisₓ) tag, a maltose binding protein (MBP) tag, a glutathione S-transferase (GST) tag, and a small ubiquitin-like modifier (SUMO) tag. In the phase transition adjusting kit of the present invention, the tag may be connected to the N-terminus and/or C-terminus of the multivalent domain.

In another aspect, the present invention provides a method for screening a phase transition adjusting kit, the method comprising the following steps:
Step A: generating a library comprising a plurality of candidate phase transition adjusting components, wherein each of the candidate phase transition adjusting components comprises a multivalent domain and comprises or dose not comprise no more than one ligand capable of specifically binding to a cell surface molecule;
Step B: arbitrarily selecting at least two of the candidate phase transition adjusting components from the library generated in step A, and combining them into a candidate phase transition adjusting kit, wherein the cell surface molecules recognized by the candidate components in the candidate kit via the ligands are the same as or different from each other; and
Step C: under conditions suitable for phase transition, measuring the activity of the candidate phase transition adjusting kit combined in step B in generating phase transition droplets, and selecting the candidate phase transition adjusting kit that generates more phase transition droplets than the negative control as the target kit, wherein the negative control is a parallel measurement not containing the candidate phase transition adjusting kit.

In another aspect, the present invention provides a phase transition adjusting kit obtained by screening through the above method for screening a phase transition adjusting kit.

In yet another aspect, the present invention provides a pharmaceutical composition, characterized in that the pharmaceutical composition comprises the aforementioned phase transition adjusting kit, and optionally a pharmaceutically acceptable carrier. In some embodiments, the pharmaceutical composition provided by the present invention is a kit.

The phase transition adjusting kit of the present invention can be combined into one preparation with one or more substances capable of inhibiting the internalization of cell surface receptors, or the kit of the present invention and the inhibitory substances for the internalization of cell surface receptors can be formulated into separate preparations so that they can be administered simultaneously, separately or sequentially. Therefore, in yet another aspect, the present invention also relates to a product comprising the phase transition adjusting kit of the present invention and one or more receptor internalization inhibitors for simultaneous, separate or sequential use as a combined preparation in the treatment of a disease.

In still another aspect, the present invention provides a method of treating a disease using any of the aforementioned phase transition adjusting kits or pharmaceutical compositions. In a preferred embodiment, the disease may be selected from the group consisting of chronic autoimmune disorders, inflammatory disorders, diseases associated with abnormal cell proliferation or abnormal cell apoptosis, sepsis or viral infection. In a more preferred embodiment, the disease associated with abnormal cell proliferation or abnormal cell apoptosis is cancer. In further preferred embodiments, the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, The invention further comprises a group consisting of carcinoma, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.

In still another aspect, the present invention provides use of the aforementioned phase transition adjusting kit or the aforementioned pharmaceutical composition in preparing a preparation for treating a disease. In a preferred embodiment, the disease may be selected from the group consisting of chronic autoimmune disorders, inflammatory disorders, diseases associated with abnormal cell proliferation or abnormal cell apoptosis, sepsis or viral infection. In a more preferred embodiment, the disease associated with abnormal cell proliferation or abnormal cell apoptosis is selected from cancer. In further preferred embodiments, the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, The invention further comprises a group consisting of carcinoma, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.

According to the present invention, a novel phase transition adjusting kit is provided, which can increase the aggregation of cell surface molecules by driving phase separation after binding to cell surface molecules, thereby, for example, changing the regulatory effect on its downstream signal transduction. Moreover, since this novel kit contains two or more different ligands, it can achieve more variable and more refined targeting specificity on the basis of combing different ligands. More specifically, the present invention provides a phase transition adjusting kit comprising at least two different recombinant ligands. Compared with each recombinant ligand itself, and compared with a combination of ligands incapable of inducing phase separation, the kit of the present invention can significantly modify the biological activity associated with the first cell surface molecule and/or the second cell surface molecule (for example, significantly changes the signal transduction effect downstream of the cell surface molecule).

### The Description of Drawings

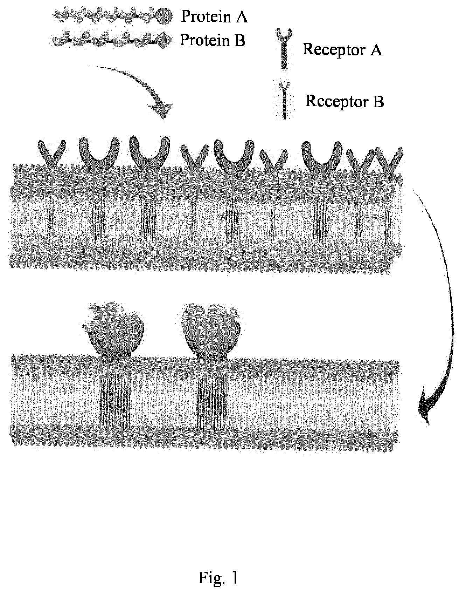

Fig. 1 is a schematic diagram of the phase transition adjusting kit of the present application for manipulating the phase separation mechanism of cell surface molecules.

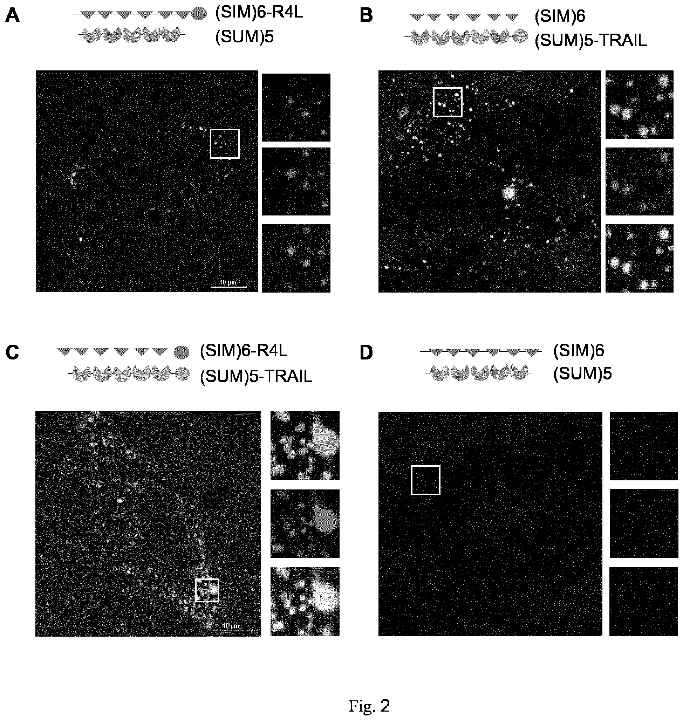

Fig. 2 is a confocal laser scanning microscopy image of human osteosarcoma cell line SJSA-1 treated with different combinations of recombinant proteins.

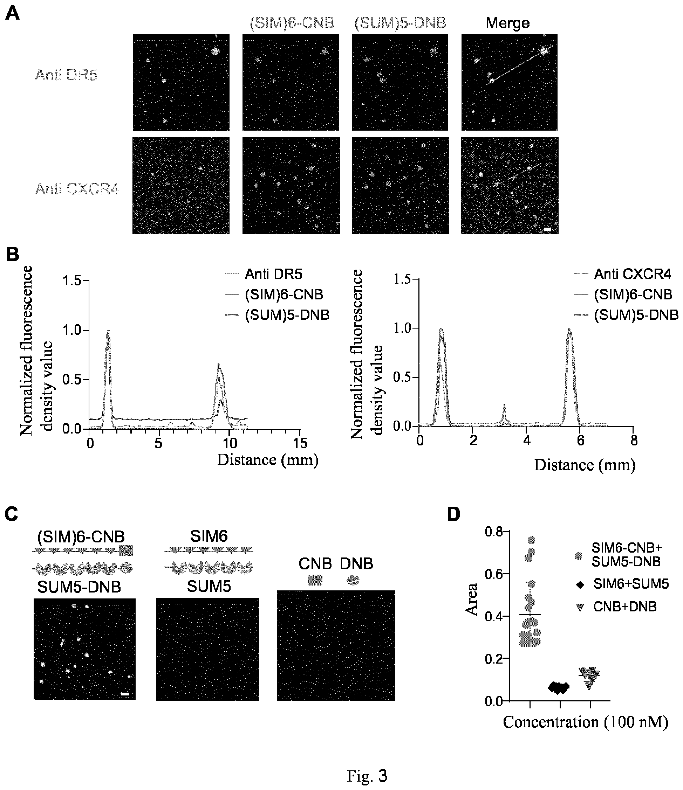

Fig. 3 shows a comparison of the results of different phase transition adjusting kits inducing phase separation in the human osteosarcoma cell line SJSA-1. Fig. 3A shows the puncta formed by the phase-change regulator and cell surface receptors after the SJSA-1 cell line was treated with (SIM)₆-CNB (red) and (SUM)₅-DNB (green), as well as the co-localization of the endogenous DR5 and CXCR4 receptors of SJSA-1 cells with the phase-change regulator in the puncta. Wherein, "Anti DR5" and "Anti CXCR4" respectively represent the fluorescence images of endogenous DR5 and CXCR4 obtained by immunofluorescence staining with DR5 antibody (Abcam, ab8416) and CXCR4 antibody (Protein Tech, 60042-1-Ig). Fig. 3B shows the fluorescence intensity analysis along the path indicated by the red line in Fig. 3A, wherein the left and right figures correspond to "merge" in the first and second rows of Fig. 3A, respectively. The horizontal axis represents the relative distance along the straight path, and the vertical axis represents the relative fluorescence intensity measured at the corresponding position. Fig. 3C shows random fields of view obtained from total internal reflection fluorescence microscopy (TIRF) imaging of live cells after SJSA-1 was treated with different protein combinations. From left to right, they correspond to treatment groups 1 to 3 listed in Example 2. Fig. 3D shows the result of fluorescence area statistics for puncta observed in the random fields of view in Fig. 3C. The horizontal axis represents various recombinant protein treatment combinations with a final concentration of 100 nM. The vertical axis represents the fluorescence area corresponding to each spot.

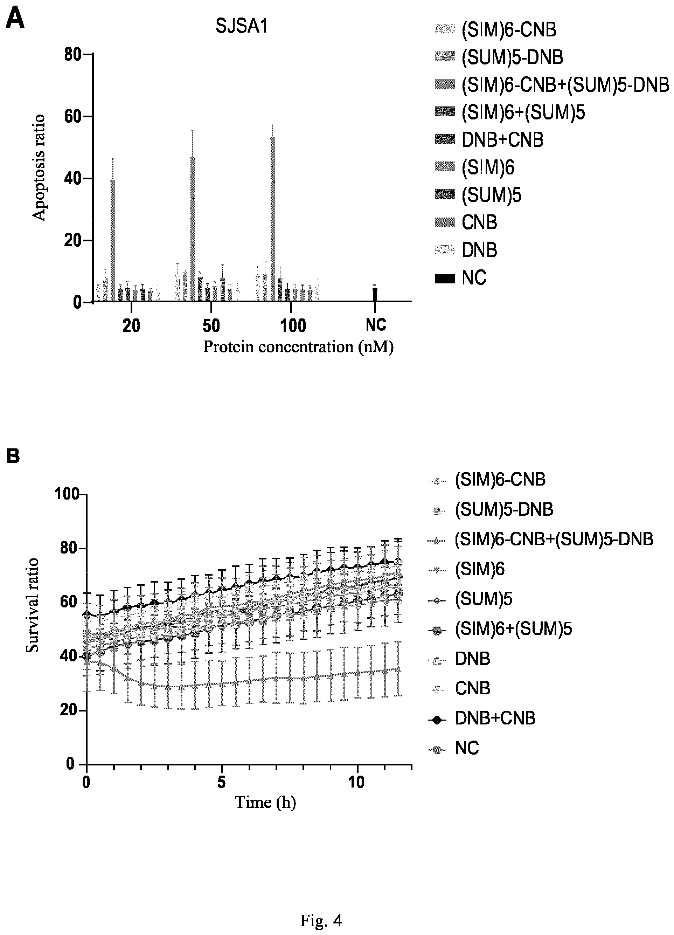

Fig. 4 shows valuation results of the apoptosis-inducing activity of different recombinant proteins or their combinations against SJSA-1 cells. Fig. 4A shows the percentage of apoptosis of SJSA-1 cells measured by flow cytometry based on Annexin V-FITC/PI double staining after different recombinant proteins or their combinations were added to the culture medium at final concentrations of 20, 50, and 100 nM and incubated for 4 hr. Fig. 4B shows the changes in the survival ratio of SJSA-1 cells over time measured by flow cytometry after adding different recombinant proteins or their combination to 100 nM. NC in the figure represents a blank control without added protein.

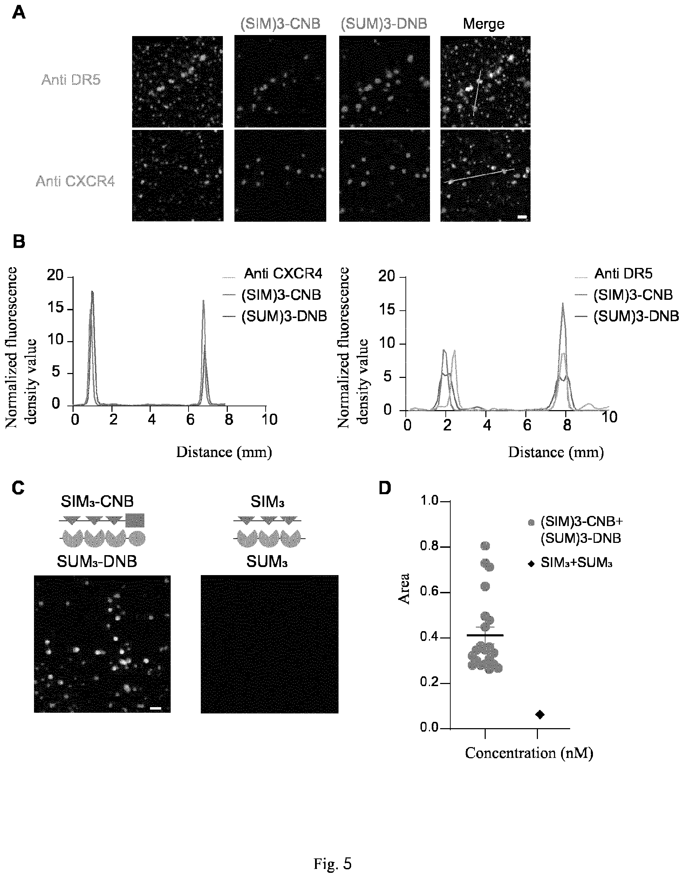

Fig. 5 shows a comparison of the phase separation of human osteosarcoma cell line SJSA-1 induced by different fusion proteins. Fig. 5A shows the puncta formed by the fusion protein and cell surface receptors after the SJSA-1 cell line was treated with (SIM)₃-CNB (Red) and (SUM)₃-DNB (Green), and the co-localization of the endogenous DR5 and CXCR4 receptors of SJSA-1 cells with the fusion protein in the puncta. Wherein, "Anti-DR5" and "Anti-CXCR4" respectively represent the fluorescence images of endogenous DR5 and CXCR4 obtained by immunofluorescence staining with DR5 antibody (Abcam, Catalog No. ab8416) and CXCR4 antibody (Protein Tech, Catalog No. 60042-1-Ig). Fig. 5B shows the fluorescence intensity analysis along the path indicated by the red line in Fig. 5A, wherein the left and right figures correspond to "merge" in the first and second rows of Fig. 5A, respectively. The horizontal axis represents the relative distance along the straight path, and the vertical axis represents the relative fluorescence intensity measured at the corresponding position. Fig. 5C shows random fields of view of TIRF imaging of live cells obtained after SJSA-1 cells were treated with the protein combination of Example 4. Fig. 5D shows the results of fluorescence area statistics for the spots observed in the random fields of view of Fig. 5C. The horizontal axis represents various recombinant protein treatment combinations with a final concentration of 100 nM. The vertical axis represents the fluorescence area corresponding to each spot.

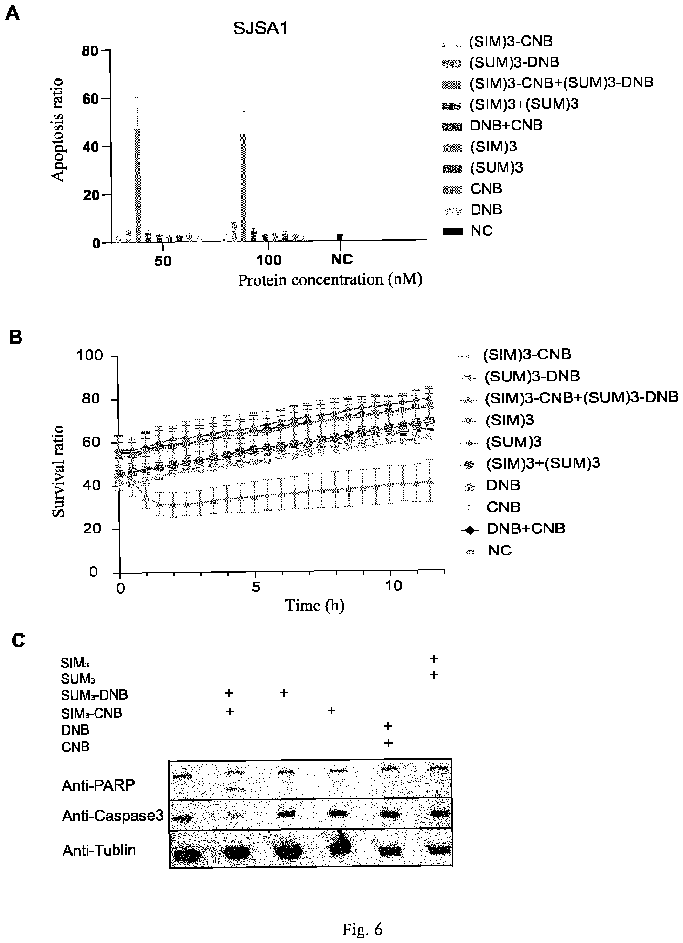

Fig. 6 shows evaluation results of apoptosis-inducing activity of different recombinant proteins or their combinations against SJSA-1 cells. Fig. 6A shows the apoptosis percentage of SJSA-1 cells measured by flow cytometry based on Annexin V-FITC/PI double staining after different recombinant proteins or their combination were added to the culture medium at final concentrations of 50 and 100 nM, respectively, and incubated for 4 hr. Fig. 6B shows the changes in the survival ratio of SJSA-1 cells over time measured by flow cytometry after adding different recombinant proteins or their combination to 100 nM. NC in the figure represents a blank control without added protein. Fig. 6C shows the results of Western immunoblotting analysis of the protein expressions of endogenous apoptosis markers PARP and Caspase 3 in SJSA-1 cells treated with 50 nM of various recombinant proteins or their combination. β-tubulin was used as a internal control.

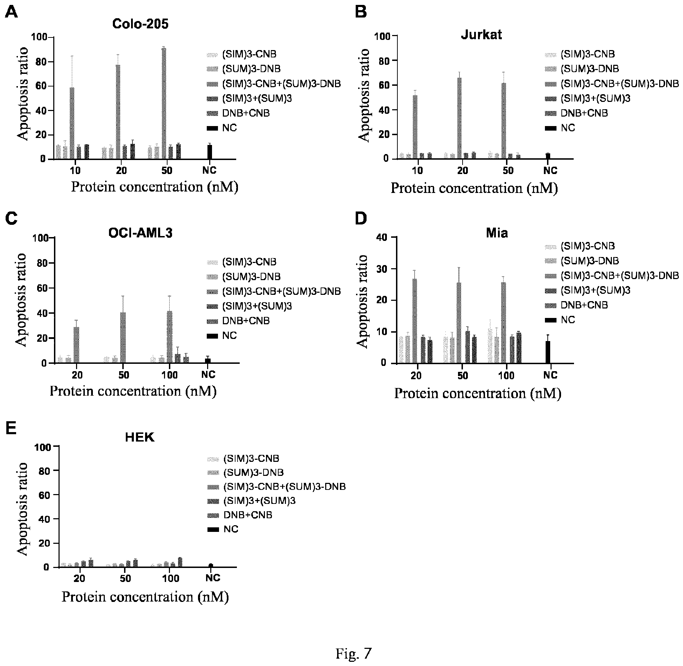

Fig. 7 shows the evaluation results of the apoptosis-inducing activity of different recombinant proteins and their combinations against five tumor cell lines and HEK293 cells. Depending on the cell lines, cells were treated with the indicated concentrations of recombinant proteins or their combinations for 3 hr or 4 hr, and then the percentage of apoptosis was determined by flow cytometry based on Annexin V-FITC/PI double staining. In the figure, NC represents a blank control without adding protein, Mia represents a pancreatic cancer cell line MIA PaCa-2, and HEK represents a human embryonic kidney cell line HEK293.

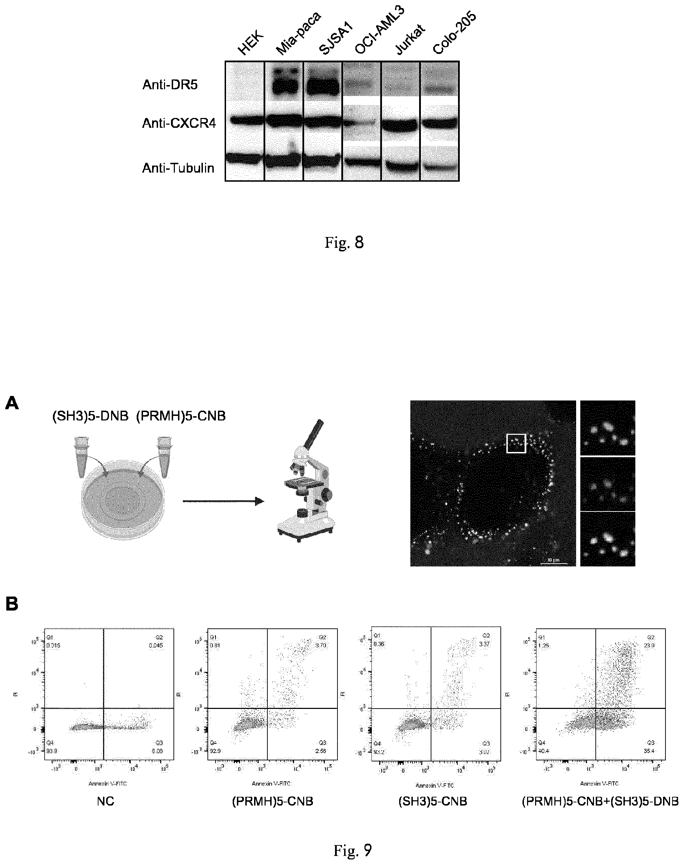

Fig. 8 shows the results of Western blotting analysis of the protein expressions of death receptor 5 (DR5) and CXC motif chemokine receptor 4 (CXCR4) in 6 different cell lines. β-tubulin was used as an internal control for calibration. HEK represents the human embryonic kidney cell line HEK293, Mia-paca represents the pancreatic cancer cell line MIA PaCa-2, OCI represents the acute myeloid leukemia cell line OCI-AML3, and Colo-205 represents the colorectal adenocarcinoma cell line COLO205.
Fig. 9 shows the manipulation of cell membrane receptors by the fusion protein of the present invention. SJSA-1 cell line was treated with (PRMH)₅-CNB and (SH3)₅-DNB and analyzed for cell apoptosis. Fig. 9A shows the results of laser confocal scanning microscopy imaging obtained by adding recombinant proteins (PRMH)₅-CNB and (SH3)₅-DNB to the human osteosarcoma cell line SJSA-1. Fig. 9B shows the apoptosis-inducing activity when recombinant proteins (PRMH)₅-CNB, (SH3)₅-DNB, or a combination of the two were added to SJSA-1 cells, and the results were compared with the negative control. Under the same conditions, SJSA-1 cells were treated with 100 nM recombinant proteins for 4 hr, and then the percentage of SJSA-1 cells distributed in different quadrants was determined by flow cytometry based on Annexin V-FITC/PI double staining. In the figure, NC represents a blank control without added protein.

### Detailed Description of the Preferred Embodiment

The present invention is further described in detail below in conjunction with specific embodiments. The given examples are only for illustrating the present invention, but not for limiting the scope of the present invention. The following examples are provided as a guide for further improvements by those skilled in the art and are not intended to limit the present invention in any way.

The experimental methods in the following examples, unless otherwise specified, are conventional methods and are performed according to the techniques or conditions described in the literature in the art or according to the product instructions. Unless otherwise specified, the materials, reagents, instruments, etc. used in the following examples can be obtained from commercial sources. The quantitative tests in the following examples, unless otherwise specified, are the average values of three repeated experiments. In the following examples, unless otherwise specified, the nucleotide sequences in the sequence table are written from left to right in the order from 5' to 3' end, and the amino acid sequences are written from left to right in the order from the amino end to the carboxyl end.

### Definition

As used in the description of the present invention, the following words and phrases are generally deemed to have the meanings set forth below, unless otherwise indicated in the context in which the words or phrases are used.

As used herein, the terms "comprise" or "include" mean that the compositions and methods include the recited elements, but do not exclude other elements. "Consisting essentially of", when used in defining compositions and methods, shall mean excluding any other components that are clearly essential to the combination. Therefore, the compositions defined herein consisting essentially of these components will not exclude trace contamination caused by separation and purification procedure or pharmaceutically acceptable carriers such as phosphate-buffered saline, preservatives and the like. "Consisting of" shall be meant to exclude minor components of other ingredients and substantial methods of administering the compositions of the present invention. Embodiments defined by these provisional terms are within the scope of the present invention.

As used herein, the term "about" refers to the common error range of the corresponding value that is readily known to those skilled in the art. A value or parameter described herein as "about" includes the value or parameter itself.

As used herein, the term "liquid-liquid phase separation" (LLPS, also referred to herein as "phase separation" or "phase transition") refers to the following transformation process occurring between multivalent macromolecules: under suitable solution conditions, multivalent macromolecules aggregate through interactions to form larger complexes, and the complexes reach corresponding solubility and separate from the common solution phase to form an independent liquid phase enriched with the complexes.

The term "phase transition droplets" refers to highly identifiable small droplets with a diameter of several microns or even larger that exist in the liquid phase formed by phase separation. In this article, "phase transition droplets" are sometimes simply referred to as "droplets".

As used herein, the term "multivalent domain" refers to a domain composed of multiple structural modules or motifs associated with phase separation. As mentioned above, biomacromolecules can aggregate and phase separate due to intermolecular or intramolecular interactions. Modules or motifs that can lead to the above-mentioned intermolecular or intramolecular interactions include, but are not limited to, (1) structural modules or motifs that are linearly arranged and have similar functions in proteins or polypeptides; (2) structural modules or motifs that promote oligomerization of proteins or polypeptides; (3) multimerization binding sites generated based on post-translational modifications; (4) intrinsically disordered regions or low-complexity domains in proteins or polypeptides (see, for example, Wang et al., Cell 174(3): 688-699, 2018; Nott, Timothy J et al., Molecular Cell 57(5): 936-947, 2015). The number of structural modules or motifs required for inducing the above-mentioned intermolecular interaction contained in the multivalent structural domain of the present invention is the valency of the multivalent structural domain. For example, for a multivalent domain (SIM)₆ consisting of six SIM motifs repeated in tandem, its valency is hexavalent.

The term "ligand" herein is used in the broadest sense to refer to the other party in a pair that has a specific interaction with each other. More specifically, the term "ligand" means a chemical entity capable of interacting with a cell surface molecule. The interaction between the ligand involved in the present invention and the cell surface molecule it targets includes covalent bonds, ionic bonds, hydrogen bonds or other bonding methods known in the art. Relative to the interacting cell surface molecule, the ligand of the present invention may be naturally occurring, such as a natural molecule isolated from a target cell population or organism from which the cell surface molecule originates, such as certain small molecule metabolites, or macromolecules such as antibodies. The ligands of the present invention may also be non-natural, such as chemical entities obtained by artificial synthesis or modification that are different from natural molecules. That is to say, the term "ligand" herein encompasses various ligand structures, including but not limited to peptide or non-peptide ligands. For example, but not limited to, various inorganic or organic small molecules, antibodies, antigen-binding fragments, peptides, peptide mimetics, antisense oligonucleotides or small interfering RNA (siRNA), as long as they show the specific binding activity required with the paired object. Preferably, the ligand herein refers to a peptide or peptide mimetic composed of natural or non-natural amino acids linked by peptide bonds, for example, an antibody or an antigen-binding fragment thereof, a cytokine, a growth factor, an adhesion molecule, a peptide hormone, and the like.

The term "cell surface molecule" herein has the meaning commonly understood in the art. Nonlimiting examples of cell surface molecules include protein molecules, sugar molecules, glycoproteins and the like, receptors such as cytokine receptors, histocompatibility receptors, T cell receptors and the like, ion channels, etc. In some cases, the cell surface molecule may be a molecule that is present in the target cell population and substantially absent (or present at a lower concentration) in other populations, thereby being able to indicate or define the type of the target cell population. In other cases, the cell surface molecule may be a molecule present in the target cell population that is directly or indirectly associated with the state or condition of the pathology to be corrected and that is substantially absent or present at lower concentrations in cells or tissues not affected by the pathology. A ligand of the present invention can bind to a cell surface through interaction with cell surface molecules, preferably to the surface of cells having a pathological state or condition to be corrected (eg, tumor cells).

Herein, "receptor" may include endogenous receptors or exogenous receptors. Endogenous receptors include receptors that occur naturally in cells. Exogenous receptors include receptors that are introduced exogenously into a cell. In some aspects, an exogenous receptor may be a naturally occurring sequence contained in other cells of the same individual. In other aspects, an exogenous receptor can be a receptor from a different organism or a different species. Exogenous receptors also include synthetic receptors that do not occur naturally in any organism. Exogenous receptors include chimeric receptors, which refer to receptors constructed by connecting regions (e.g., extracellular, transmembrane, intracellular, etc. domains) of different molecules (e.g., different proteins, homologous proteins, orthologous proteins, etc.).

Herein, "tandemly linked" includes directly connecting the connection units through covalent bonds or indirectly connecting them through spacer sequences (also called linkers, connection sequences) and the like. In the tandem linked structure, the connection units can be arranged in the same direction or in different directions.

The term "increase" or "activate" as used herein means the ability to cause an overall increase, e.g., an overall increase of 10% or more, 20% or more, 30% or more, 40% or more, 50% or more, or 75%, 85%, 90%, 95% or more. In certain aspects, increase or activation can refer to downstream activity of a ligand-cell surface molecule interaction.

The terms "reduce" or "inhibit" as used herein refer to the ability to cause an overall decrease, e.g., an overall decrease of 10% or more, 20% or more, 30% or more, 40% or more, 50% or more, or 75%, 85%, 90%, 95% or more. In certain aspects, reduction or inhibition can refer to an activity downstream of a ligand-cell surface molecule interaction.

As used herein, the term "oligomerization" means that several biomacromolecules, such as several receptor molecules, are aggregated into a complex by non-covalent bonds, and the functional state may be changed.

The term "antibody" herein encompasses various antibody structures including, but not limited to, monoclonal antibodies, polyclonal antibodies, multispecific antibodies (e.g., bispecific antibodies), and antibody fragments (e.g., bis-Fab).

The term "intact antibody" is used to refer to an antibody having a structure substantially similar to a native antibody structure or having heavy chains containing an Fc region as defined herein. Herein, "intact antibody" is used interchangeably with "full length antibody" and "whole antibody".

The term "antigen-binding fragment" or "antibody fragment" refers to a molecule other than an intact antibody that comprises a portion of an intact antibody that binds to the antigen to which the intact antibody binds. Examples of antigen-binding fragments include, but are not limited to, bis-Fab, Fv, Fab, Fab, Fab'-SH, F(ab')₂, diabodies, linear antibodies, single-chain antibodies (e.g., scFv, ScFab), single-domain antibodies (e.g., VH domain, VHH domain or nanobody), and multispecific antibodies formed from antibody fragments.

The term "single-chain antibody", also called "single-chain Fv", "single-chain variable fragment", "sFv" or "scFv", are antibody fragments comprising the VH and VL antibody domains connected in a single polypeptide chain. Preferably, the scFv polypeptide further comprises a polypeptide linker between the VH and VL domains to enable the scFv to form a desired antigen binding structure. For a review of scFv, see Pluckthun, The Pharmacology of Monoclonal Antibodies, Vol. 113, Rosenburg and Moore, eds., Springer Verlag, New York, pp. 269-315 (1994); Malmborg et al., J. Immunol. Methods 183:7-13, 1995.

The term "single domain antibody" refers to an antibody fragment comprising all or part of the heavy chain variable domain or all or part of the light chain variable domain of an antibody. In certain aspects, the single domain antibody is a human single domain antibody (see, e.g., US Pat. No. 6,248,516 Bl). Examples of single domain antibodies include, but are not limited to, VHHs.

The term "small molecule" refers to any molecule having a molecular weight of about 2000 Daltons or less, such as about 1000 Daltons or less. In some aspects, the small molecule can be an organic molecule. In other aspects, the small molecule can be an inorganic molecule.

As used herein, the term "mimetic" or "molecular mimetic" refers to a polypeptide that has sufficient similarity in conformation and/or binding ability (e.g., secondary structure, tertiary structure) to a given polypeptide or a portion of said polypeptide to bind to the binding partner of said polypeptide. A mimetic may bind to a binding partner with equal, lesser, or greater affinity than the polypeptide it mimics. A molecular mimetic may or may not have significant amino acid sequence similarity to the polypeptide it mimics. Mimetics can be naturally occurring or engineered. In some aspects, a mimetic can perform all of the functions of the polypeptide being mimicked. In other aspects, a mimetic does not perform all of the functions of the polypeptide it is mimicking.

As used herein, "tumor-associated antigen" or "TAA" refers to an antigenic determinant present on the surface of a target cell, eg, a cell in a tumor (such as a cancer cell, a cell in tumor matrix). In certain aspects, the target cell antigen is an antigen on the surface of a tumor cell. In one aspect, the TAA is selected from the group consisting of: CXC motif chemokine receptor 4 (CXCR4), hepatocyte growth factor receptor (c-Met or HGFR), epidermal growth factor receptor (EGFR), epidermal growth factor receptor 2 (HER2) and prostate specific membrane antigen (PSMA), fibroblast activation protein (FAP), carcinoembryonic antigen (CEA), folate receptor alpha (FolR1), melanoma-associated chondroitin sulfate proteoglycan (MCSP), p95HER2, EpCAM, HER3, CD30 or TPBG (5T4), CD19, CD79b, CD20, CD22, CD37, CD38, BCMA and GPRC5D.

Unless otherwise indicated, the term "CXC motif chemokine receptor 4" or "CXCR4" as used herein refers broadly to any native CXCR4 from any vertebrate source, including mammals such as primates (e.g., humans), non-human primates (e.g., cynomolgus monkeys), and rodents (e.g., mice and rats). The term encompasses full-length CXCR4 and isolated regions or domains of CXCR4, such as the extracellular domain of CXCR4. The term also encompasses naturally occurring variants of CXCR4, such as splice variants or allelic variants. An exemplary amino acid sequence of human CXCR4 is shown in Uniprot ID: P61073. The present invention also contemplates minor sequence variations, particularly conservative amino acid substitutions of CXCR4 that do not affect the function and/or activity of CXCR4.

Unless otherwise indicated, the term "death receptor 5" or "DR5" as used herein refers broadly to any native DR5 from any vertebrate source, including mammals such as primates (e.g., humans), non-human primates (e.g., cynomolgus monkeys), and rodents (e.g., mice and rats). The term encompasses full-length DR5 and isolated regions or domains of DR5, such as a DR5 extracellular domain. The term also encompasses naturally occurring variants of DR5, such as splice variants or allelic variants. An exemplary amino acid sequence of human DR5 is shown in Uniprot ID: 014763. The present invention also contemplates minor sequence variations, especially conservative amino acid substitutions of DR5 that do not affect the function and/or activity of DR5.

The term "epidermal growth factor receptor (EGFR)" is also known as proto-oncogene c-ErbB-1 or receptor tyrosine protein kinase erbB-1. Unless otherwise indicated, the term refers broadly to any native EGFR from any vertebrate source, including mammals such as primates (eg, humans), non-human primates (e.g., cynomolgus monkeys), and rodents (e.g., mice and rats). The term encompasses full-length EGFR and isolated regions or domains of EGFR, such as the EGFR extracellular domain. The term also encompasses naturally occurring variants of EGFR, such as splice variants or allelic variants. The amino acid sequence of human EGFR is shown in UniProt Accession No. P00533 (version 211). The present invention also contemplates minor sequence variations, especially conservative amino acid substitutions of EGFR that do not affect the function and/or activity of EGFR.

Unless otherwise indicated, the term "hepatocyte growth factor receptor (Met)" as used herein refers broadly to any native hepatocyte growth factor receptor from any vertebrate source, including mammals such as primates (e.g., humans), non-human primates (e.g., cynomolgus monkeys), and rodents (e.g., mice and rats). The term encompasses the full-length hepatocyte growth factor receptor as well as isolated regions or domains thereof, such as the extracellular domain of the hepatocyte growth factor receptor. The term also encompasses naturally occurring variants of the hepatocyte growth factor receptor, such as splice variants or allelic variants. The present invention also contemplates minor sequence variations, especially conservative amino acid substitutions of the hepatocyte growth factor receptor that do not affect the function and/or activity of the hepatocyte growth factor receptor.

As used herein, "internalization" refers to the transfer of a cell surface protein to the interior of a cell through the process of internalization. Therefore, "receptor internalization inhibitor" refers to an agent that can inhibit, hinder, reduce or eliminate the receptor internalization process, including but not limited to any substance known in the art or not yet found to have internalization inhibitory activity. Receptor internalization inhibitors affect signal transduction and cell function by interfering with or hindering the internalization process of receptors and their bound signal molecules.

As used herein, "disease" refers to any condition that would benefit from treatment, including, but not limited to, chronic and acute disorders or diseases, including those pathological conditions that predispose a mammal to the disorder. In certain aspects, the disease is a disease associated with altered function or activity of a cell surface molecule. In certain preferred aspects, the disease is a chronic autoimmune disorder, an inflammatory disorder, a disease associated with abnormal cell proliferation, a disease associated with abnormal cell apoptosis, sepsis, or a viral infection. In the most preferred aspect, the disease is cancer.

The term "cancer" as used herein refers to or describes the physiological condition in mammals that is typically characterized by uncontrolled cell growth/proliferation. Cancer aspects include solid tumor cancers and non-solid tumor cancers. Solid cancer tumors include, but are not limited to, lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid carcinoma), or prostate cancer, or metastatic forms thereof. In some aspects, the cancer is colorectal cancer (CRC). In some aspects, the cancer is lung cancer. Further aspects of lung cancer include epidermal growth factor receptor positive (EGFR⁺) lung cancer. Other aspects of lung cancer include non-small cell lung cancer (such as squamous lung cancer or non-squamous lung cancer) and small cell lung cancer. In some aspects, the cancer is prostate cancer. Further aspects of prostate cancer include castration-resistant prostate cancer (CRPC). In some aspects, the cancer is breast cancer. Further aspects of breast cancer include HER2-positive (HER2⁺) breast cancer. Other aspects of breast cancer include ductal carcinoma. In some aspects, the breast cancer is early stage breast cancer. In some aspects, the cancer is a metastatic form of a solid tumor. In some aspects, the metastatic form of a solid tumor is a metastatic form of lung cancer, colorectal cancer, head and neck cancer, glioma, neuroblastoma, melanoma, breast cancer, bladder cancer, kidney cancer, ovarian cancer, pancreatic cancer, cervical cancer, esophageal cancer, sarcoma, esophageal cancer, thyroid cancer, and prostate cancer. In some aspects, the cancer is a non-solid tumor cancer. Non-solid tumor cancers include, but are not limited to, B-cell lymphomas. Further aspects of B-cell lymphoma include, for example, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt lymphoma or mycosis fungoides (MF).

The term "pharmaceutically acceptable carrier" refers to a non-toxic carrier that can be administered to a patient together with the phase-transition adjusting element of the present invention without destroying the functional activity of the regulatory element. In certain embodiments, a "pharmaceutically acceptable" substance is suitable for use in contact with cells, tissues or organs of animals or humans without excessive toxicity, irritation, allergic response, immunogenicity or other adverse reaction, and the amount used in the dosage form is proportional to a reasonable benefit/risk ratio according to the administration schedule. In certain embodiments, a "pharmaceutically acceptable" substance as a component of a pharmaceutical composition is also compatible with the other ingredients of the composition. In certain embodiments, the term "pharmaceutically acceptable carrier" includes, but is not limited to, pharmaceutically acceptable inactive ingredients, materials, compositions and vehicles, such as liquid fillers, solid fillers, diluents, excipients, carriers, solvents and encapsulating materials. Carriers also include all pharmaceutically acceptable dispersion media, coatings, buffers, isotonic agents, stabilizers, absorption delaying agents, antimicrobial agents, antibacterial agents, antifungal agents, adjuvants, and the like. Unless any conventional carrier is incompatible with the phase transition adjusting element, the present disclosure contemplates use of conventional carriers in pharmaceutical compositions. See, e.g., Remington: The Science and Practice of Pharmacy, 21st ed., Lippincott Williams & Wilkins (Philadelphia, Pennsylvania, 2005); Handbook of Pharmaceutical Excipients, 5th Ed., Rowe et al., ed., The Pharmaceutical Press and the American Pharmaceutical Association (2005); Handbook of Pharmaceutical Additives, 3rd ed., Ash and Ash, ed., Gower Publishing Co. (2007); and Pharmaceutical Preformulation and Formulation, Gibson, ed., CRC Press LLC (Boca Raton, Florida, 2004).

In addition, the PCT international application entitled "A phase change adjusting element and use thereof" filed on the same date as the present application is also incorporated herein by reference in its entirety.

The following describes the preferred modes for implementing the present invention. It should be noted that the embodiments described below are examples showing typical embodiments of the present invention, but the present invention is not limited to these examples.

Two or more of the embodiments described below may be combined, and such a combination is also included in the present invention.

### Examples

### Example 1

It is known that the chemokine receptor CXCR4 is located on the cell membrane and can bind to its ligand R4L; the death receptor DR5 is also located on the cell membrane and can interact with its ligand TRAIL.

By using a DNA cloning method commonly known in the art (Dhoble et al., International Journal of Genetic Engineering and Recombination 3.1: 1-10, 2017), DNA fragments encoding the following tandemly linked motifs were constructed: a multivalent domain (SUM)₅ obtained by tandemly connecting five SUMO3 motifs, and a multivalent domain (SIM)₆ obtained by tandemly connecting six SIM motifs. Then, the DNA fragment encoding R4L was connected with the DNA fragment encoding (SIM)₆ to construct the recombinant vector pRSFDuet-1-(SIM)₆-R4L. The DNA fragment encoding TRAIL was ligated to the coding region encoding (SUM)₅ to construct the recombinant vector pRSFDuet-1-(SUM)₅-TRAIL. By using the abovementioned recombinant vectors as well as the prokaryotic expression and purification methods generally known in the art, recombinant ligands (SIM)₆-R4L and (SUM)₅-TRAIL which fused with multivalent domains were prepared. The specific steps are as follows:

### 1. Construction of recombinant vectors

**(SIM)₆ expression vector:** the DNA fragment shown in SEQ ID NO: 1 was synthesized by a DNA fragment synthesis method known in the art (Kosuri et al., Nature methods 11(5): 499-507, 2014). PCR amplification was performed using the primer pair (SIM)₆-F and (SIM)₆-R with the DNA fragment shown in SEQ ID NO: 1 as a template:
(SIM)₆-F:
   ACTTTAATAAGGAGATATACCATGAAACATCATCATCATCA (SEQ ID NO: 29)
(SIM)₆-R:
   TTCTTTACCAGACTCGAGTTACCCCATGGAGCCGCCGCTA (SEQ ID NO: 30).

PCR amplification was performed for 30 cycles as follows: denaturation at 94°C for 0.5 min, annealing at 60°C for 0.5 min, and extension at 72°C for 1 min. The obtained PCR product and vector pRSFDuet-1 were then digested with restriction enzymes NcoI and XhoI, and the purified PCR product fragment and vector fragment were ligated with T4 ligase to obtain a recombinant vector, wherein the recombinant vector verified to have correct sequence was recorded as pRSFDuet-1-(SIM)₆. Therefore, pRSFDuet-1-(SIM)₆ can express the multivalent domain (SIM)₆ shown in SEQ ID NO: 2 (hereinafter, this domain will also be referred to as (SIM)₆).

For ease of understanding, in SEQ ID NO: 1, positions 7-27 encode 7×HisTag, positions 28-48 encode TEV, and positions 61-735 encode (SIM)₆. The DNA sequence shown in SEQ ID NO: 1 encodes the protein shown in SEQ ID NO: 2. Positions 21-245 in SEQ ID NO: 2 are the amino acid sequence of the multivalent domain (SIM), and positions 21-43 are the amino acid sequence of the monovalent SIM (i.e., a single SIM motif).

**(SUM)₅ expression vector:** by the same cloning method as above, using the primer pair (SUM)₆-F and (SUM)₆-R shown below, PCR amplification was performed with the DNA fragment shown in SEQ ID NO: 3 as a template to obtain the recombinant vector pRSFDuet-1-(SUM)₅.
(SUM)₆-F:
   ACTTTAATAAGGAGATATACCATGAAACATCATCATCATCATC (SEQ ID NO: 31),
(SUM)₆-R:
   TTCTTTACCAGACTCGAGTTAGCCCATGGAGCCGCCGCTA (SEQ ID NO: 32).

pRSFDuet-1-(SUM)₅ can express the multivalent domain (SUM)₅ shown in SEQ ID NO: 4 (hereinafter, the domain will also be referred to as (SUM)₅).

For ease of understanding, in SEQ ID NO: 3, positions 7-27 encode 7×HisTag, positions 28-48 encode TEV, and positions 79-1653 encode (SUM)₅. The DNA sequence shown in SEQ ID NO: 3 encodes the protein shown in SEQ ID NO: 4, wherein positions 27-551 in sequence 4 are the amino acid sequence of the multivalent domain (SUM)₅, and positions 27-118 are the amino acid sequence of the monovalent SUMO₃ (i.e., a single SUMO₃ motif).

**(SIM)₆-R4L expression vector:** Using the same cloning method as above, using the primer pair (SIM)₆-R4L-F and (SIM)₆-R4L-R shown below, PCR amplification was performed with the DNA fragment shown in SEQ ID NO: 5 as a template to obtain the recombinant vector pRSFDuet-1-(SIM)₆-R4L.
(SIM)₆-R4L-F:
   ACTTTAATAAGGAGATATACCATGAAACATCATCATCATCAT (SEQ ID NO: 33),
(SIM)₆-R4L-R;
   TTCTTTACCAGACTCGAGTTAAGAACCACCAGAACCACC (SEQ ID NO: 34).

pRSFDuet-1-(SIM)₆-R4L can express the fusion protein shown in SEQ ID NO: 6, hereinafter also referred to as recombinant protein (SIM)₆-R4L.

For ease of understanding, in SEQ ID NO: 5, positions 7-27 encode 7×HisTag, positions 28-48 encode TEV, positions 61-735 encode (SIM)₆, and positions 736-822 encode R4L. The DNA sequence shown in SEQ ID NO: 5 encodes the protein shown in SEQ ID NO: 6. In SEQ ID NO: 6, positions 21-245 are the amino acid sequence of (SIM)₆, and positions 246-274 are the amino acid sequence of R4L.

**(SUM)₅-TRAIL expression vector:** Using the same cloning method as above, using the primer pair (SUM)₅-TRAIL-F and (SUM)₅-TRAIL-R shown below, PCR amplification was performed with the DNA fragment shown in SEQ ID NO: 7 as a template to obtain the recombinant vector pRSFDuet-1-(SUM)₅-TRAIL.
(SUM)₅-TRAIL-F:
   ACTTTAATAAGGAGATATACCATGAAACATCATCATCATCATC (SEQ ID NO: 35)
(SUM)₅-TRAIL-R:
   TTCTTTACCAGACTCGAGttaGCCAACCAGGAACGCACC (SEQ ID NO: 36).

pRSFDuet-1-(SUM)₅-TRAIL can express the fusion protein shown in SEQ ID NO: 8, hereinafter also referred to as recombinant protein (SUM)₅-TRAIL.

For ease of understanding, in SEQ ID NO: 7, positions 7-27 encode 7×HisTag, positions 28-48 encode TEV, positions 79-1653 encode (SUM)₅, and positions 1654-2127 encode TRAIL. The DNA sequence shown in SEQ ID NO: 7 encodes the protein shown in SEQ ID NO: 8. In SEQ ID NO: 8, positions 27-551 are the amino acid sequence of (SUM)₅, and positions 552-719 are the amino acid sequence of TRAIL.

### 2. Protein Expression and Purification

The recombinant vectors pRSFDuet-1-(SIM)₆, pRSFDuet-1-(SUM)₅, pRSFDuet-1-(SIM)₆-R4L and pRSFDuet-1-(SUM)₅-TRAIL obtained in the above step "1. Construction of recombinant vectors" were used to express the target recombinant proteins (SIM)₆, (SUM)₅, (SIM)₆-R4L and (SUM)₅-TRAIL, respectively, in the following steps:
The constructed recombinant vector was used to transfect Escherichia coli BL21. The colonies were picked up the next day and transferred to 50 mL of LB medium with kanamycin (Kan) resistance (Luria-Bertani medium: 10 g/L tryptone, 5 g/L yeast extract, 10 g/L sodium chloride), cultured at 37°C, 220 rpm for 4 h, and then transferred to 1 L of LB medium containing kanamycin (100 µg/ml concentration) and expanded at 37°C, 220 rpm.

When the bacterial concentration reaches OD₆₀₀ = 0.8~1, cool to 18°C, add 1 ml of 1 mM IPTG and let stand overnight to induce recombinant protein expression. After 17 hours of induction, the cells were enriched and disrupted by ultrasound. Then, centrifuge at 20,000 × g and 4°C for 1 hr. The centrifuged supernatant was subjected to affinity chromatography and gel filtration chromatography in sequence, and the collected protein samples were identified by SDS-PAGE to obtain the target recombinant proteins (SIM)₆-R4L, (SUM)₅-TRAIL, (SIM)₆, and (SUM)₅.

### 3. Cell Treatment

According to the manufacturer's instructions, the recombinant proteins (SIM)₆-R4L and (SIM)₆ obtained in the above "2. Protein expression and purification" were labeled with Alexa594 (ThermoFisher, A20004)-Red. The obtained Alexa594-Red labeled proteins were respectively designated as (SIM)₆-R4L (Red) and (SIM)₆ (Red). Similarly, the recombinant proteins (SUM)₅-TRAIL and (SUM)₅ were labeled with Alexa647 (ThermoFisher, A20006)-Green, and the obtained Alexa647-Green labeled proteins were designated as (SUM)₅-TRAIL (Green) and (SUM)₅ (Green), respectively.

Human osteosarcoma SJSA-1 cells (purchased from ATCC (https://www.atcc.org/)) were treated with the above-labeled recombinant proteins or control solution, and then immediately subjected to laser confocal scanning microscopy imaging. The steps are as follows:
Human osteosarcoma cell line SJSA-1 was cultured in RMPI-1640 medium containing 10% fetal bovine serum (FBS, purchased from Gibco) and 1X penicillin-streptomycin (purchased from HyClone) to 70%-80% confluence. The following combinations of recombinant proteins were added to the culture medium at the same time: (1) (SIM)₆-R4L (Red) and (SUM)₅ (Green), (2) (SIM)₆ (Red) and (SUM)₅-TRAIL (Green), (3) (SIM)₆-R4L (Red) and (SUM)₅-TRAIL (Green), (4) (SIM)₆ (Red) and (SUM)₅ (Green), to a final concentration of 100 nM respectively. The culture medium after adding the recombinant protein was mixed, incubated at 37°C for about 1 min, and then imaged using a NIKON A1R HD25 laser confocal microscope (purchased from Nikon, Inc.). The results are shown in Fig. 2.

The results showed that when the specific ligand molecules of the cell surface receptor were fused to either or both of the multivalent domains (SIM)₆ and (SUM)₅, in the presence of both the multivalent SUMO3 motif and the multivalent SIM motif, the red fluorescent signal (Red) labeled with the multivalent SIM motif and the green fluorescent signal (Green) labeled with the SUMO3 motif could co-localize on the cell membrane and show obvious clustered distribution spots (puncta) (see Fig. 2A to Fig. 2C). Furthermore, the number of spots when the two multivalent domains were fused with different ligand molecules (Fig. 2C) was significantly more than that when they were only fused with the same ligand molecule (Figs. 2A and 2B). On the other hand, the above-mentioned clustered puncta were not observed in the control group (see Fig. 2D).

The above results indicate that the multivalent domain itself cannot induce cell surface phase separation, but when it is fused with the specific ligand molecule of the cell surface receptor, the multivalent domain interacts with the cell surface receptor protein through the specific ligand, which can further increase the local concentration of the multivalent domain components and/or receptor proteins, thereby promoting the emergence of phase separation.

### Example 2

Fusion proteins were constructed by the same method as in Example 1, using nanobody CXCR4 nanobody (also referred to herein as CNB) instead of R4L and nanobody DR5 nanobody (also referred to herein as DNB) instead of TRAIL, and the induced phase separation experiments were performed in the same manner.

### 1. Construction of recombinant vectors

**(SIM)₆-CNB expression vector:** PCR amplification was performed using primer pairs (SIM)₆-CNB-F and (SIM)₆-CNB-R and the DNA fragment shown in SEQ ID NO: 9 as a template to obtain the recombinant vector pRSFDuet-1-(SIM)₆-CNB.
(SIM)₆-CNB-F:
   ACTTTAATAAGGAGATATACCATGAAACATCATCATCATCATC (SEQ ID NO: 37),
(SIM)₆-CNB-R;
   TTCTTTACCAGACTCGAGTTAAGAGCTTACGGTAACCTGA (SEQ ID NO: 38).

pRSFDuet-1-(SIM)₆-CNB can express the fusion protein shown in SEQ ID NO: 10, which is also referred to as recombinant protein (SIM)₆-CNB hereinafter.

For ease of understanding, in SEQ ID NO: 9, positions 7-27 encode the DNA sequence encoding 7×HisTag, positions 28-48 encode TEV, positions 61-735 encode (SIM)₆, and positions 736-1119 encode CNB. The DNA sequence shown in SEQ ID NO: 9 encodes the protein shown in SEQ ID NO: 10. In SEQ ID NO: 10, positions 21-245 are the amino acid sequence of (SIM)₆, and positions 246-373 are the amino acid sequence of CNB.

**(SUM)₅-DNB expression vector:** PCR amplification was performed using primer pair (SUM)₅-DNB-F and (SUM)₅-DNB-R and the DNA fragment shown in SEQ ID NO: 11 as a template to obtain the recombinant vector pRSFDuet-1-(SUM)₅-DNB.
(SUM)₅-DNB-F:
   ACTTTAATAAGGAGATATACCATGAAACATCATCATCATCAT (SEQ ID NO: 39)
(SUM)₅-DNB-R:
   TTCTTTACCAGACTCGAGTTAAGAGCTTACGGTAACGAG (SEQ ID NO: 40).

pRSFDuet-1-(SUM)₅-DNB can express the fusion protein shown in SEQ ID NO: 12, hereinafter also referred to as recombinant protein (SUM)₅-DNB.

For ease of understanding, in SEQ ID NO: 11, positions 7-27 encode 7×HisTag, positions 28-48 encode TEV, positions 79-1653 encode (SUM)₅, and positions 1654-2019 encode DNB. The DNA sequence shown in SEQ ID NO: 11 encodes the protein shown in SEQ ID NO: 12, wherein positions 27-551 in SEQ ID NO: 12 are the amino acid sequence of (SUM)₅, and positions 552-673 are the amino acid sequence of DNB.

**CNB expression vector:** PCR amplification was performed using primer pairs pRSFDuet-1-CNB-F and pRSFDuet-1-CNB-R with the DNA fragment shown in SEQ ID NO: 13 as a template to obtain the recombinant vector pRSFDuet-1-CNB.
pRSFDuet-1-CNB-F:
   ACTTTAATAAGGAGATATACCATGAAACATCATCATCATCAT (SEQ ID NO: 41),
pRSFDuet-1-CNB-R;
   TTCTTTACCAGACTCGAGTTAAGAGCTTACGGTAACCTG (SEQ ID NO: 42).

pRSFDuet-1-CNB can express the fusion protein shown in SEQ ID NO: 14, hereinafter also referred to as recombinant protein CNB.

For ease of understanding, in SEQ ID NO: 13, positions 7-27 encode 7×HisTag, positions 28-48 encode TEV, and positions 61-444 encode CNB. The DNA sequence shown in SEQ ID NO: 13 encodes the protein shown in SEQ ID NO: 14, and positions 21-148 in SEQ ID NO: 14 are the amino acid sequence of CNB.

**DNB expression vector:** Use primer pair pRSFDuet-1-DNB-F and pRSFDuet-1-DNB-R to perform PCR amplification with the DNA fragment shown in SEQ ID NO: 15 as a template to obtain the recombinant vector pRSFDuet-1-DNB.
pRSFDuet-1-DNB-F:
   ACTTTAATAAGGAGATATACCATGAAACATCATCATCATCAT (SEQ ID NO: 43)
pRSFDuet-1-DNB-R:
   TTCTTTACCAGACTCGAGTTAAGAGCTTACGGTAACGAGA (SEQ ID NO: 44).

pRSFDuet-1-DNB can express the fusion protein shown in SEQ ID NO: 16, hereinafter also referred to as recombinant protein DNB.

For ease of understanding, in SEQ ID NO: 15, positions 7-27 encode 7×HisTag, positions 28-48 encode TEV, and positions 79-444 encode DNB. The DNA sequence shown in SEQ ID NO: 15 encodes the protein shown in SEQ ID NO: 16, and positions 27-148 in SEQ ID NO: 16 are the amino acid sequence of DNB.

### 2. Protein Expression and Purification

The target recombinant protein was expressed using the recombinant vectors pRSFDuet-1-(SIM)₆-CNB, pRSFDuet-1-(SUM)₅-DNB, pRSFDuet-1-CNB and pRSFDuet-1-DNB obtained in "1. Construction of recombinant vectors" of this example. By the same purification method as in Example 1, the target recombinant proteins (SIM)₆-CNB, (SUM)₅-DNB, CNB and DNB were obtained.

### 3. Cell Treatment

According to the manufacturer's instructions, the recombinant proteins (SIM)₆-CNB and CNB obtained in the above "2. Protein expression and purification" were labeled with Alexa594 (ThermoFisher, A20004)-Red. The obtained Alexa594-Red labeled proteins were respectively designated as (SIM)₆-CNB (Red) and CNB (Red). Similarly, the recombinant proteins (SUM)₅-DNB and DNB were labeled with Alexa647 (ThermoFisher, A20006)-Green, and the resulting Alexa647-Green labeled proteins were denoted as (SUM)₅-DNB (Green) and DNB (Green), respectively.

By the same method as in Example 1, laser confocal scanning microscopy imaging was performed on human osteosarcoma cells SJSA-1 treated with the following recombinant proteins:
Treatment Group 1: combination of (SIM)₆-CNB (Red) and (SUM)₅-DNB (Green),
Treatment Group 2: combination of (SIM)₆ (Red) and (SUM)₅ (Green),
Treatment group 3: combination of CNB (Red) and DNB (Green).

The above recombinant protein combinations were added to the culture medium to a final concentration of 100 nM. The added culture medium was mixed, incubated at 37°C for about 1 min, and then imaged by total internal reflection fluorescence microscopy (TIRF). The results are shown in Fig. 3.

Figs. 3A and 3B show that co-localization of red and green fluorescence was observed in treatment group 1, indicating that the added recombinant proteins (SIM)₆-CNB (Red) and (SUM)₅-DNB (Green) aggregated on the cell membrane and formed puncta that are characteristic of liquid-liquid phase separation. Figs. 3A and 3B also show that endogenous receptors DR5 and CXCR4 expressed by cells co-localize with the added recombinant proteins in phase-separated spots.

Meanwhile, Figs. 3C and 3D show that, in Treatment Group 2, no phase separation spots were observed at all. In treatment group 3, although smaller and atypical spot structures could be detected, the fluorescent signals in these spots were weak and there was a lack of co-localization between the two fluorescent signals. The above results indicate that the use of multivalent domains or ligands alone cannot induce phase separation on the cell surface, and the emergence of phase separation requires the involvement of both sides.

### Example 3

The apoptosis-inducing effects of the recombinant proteins obtained in Examples 1 and 2 and their combination were determined by flow cytometry. The assay was performed using the human osteosarcoma cell line SJSA-1. The specific steps are as follows:
SJSA-1 cells were cultured in RPMI 1640 medium (containing 10% FBS and 1% penicillin-streptomycin) to a density of 10⁶ cells/ml. The following recombinant proteins and controls were added to the culture medium of SJSA-1 cells to a final concentration of 20, 50, and 100 nM:
(SIM)₆-CNB (Group 1), (SUM)₅-DNB (Group 2), combination of (SIM)₆-CNB and (SUM)₅-DNB (Group 3), combination of (SIM)₆ and (SUM)₅ (Group 4), combination of DNB and CNB (Group 5), (SIM)₆ (Group 6), (SUM)₅ (Group 7), CNB (Group 8), DNB (Group 9), negative control (Group 10).

After incubation at 37°C for 4 h, the cells were collected and centrifuged at 1700 rpm for 5 min, and the supernatant was removed. The precipitated cell pellet was resuspended in 500 µL PBS, centrifuged again for 5 min, and the supernatant was removed. According to the manufacturer's instructions, Annexin V-FITC/PI double staining was performed using the Annexin V-FITC cell apoptosis detection kit (purchased from Beyotime), and the cell apoptosis status was detected using the flow cytometer LSRF ortessa SORP (BD) and statistical analysis was performed. The apoptotic cell ratio is the relative ratio of the number of apoptotic cells to the total number of cells. The results are shown in Table 1 and Fig. 4A.

**Table 1. Average apoptosis ratio of SJSA-1 cells (%)***

| Groups | Recombinant protein or combination thereof | 20 nM | 50 nM | 100 nM |
|---|---|---|---|---|
| 1 | (SIM)₆-CNB | 6.23 | 8.90 | 8.53 |
| 2 | (SUM)₅-DNB | 7.71 | 9.70 | 9.12 |
| 3 | (SIM)₆-CNB + (SUM)₅-DNB | 39.55 | 46.87 | 53.31 |
| 4 | (SIM)₆ + (SUM)₅ | 4.19 | 8.13 | 7.93 |
| 5 | DNB+CNB | 4.48 | 4.60 | 4.18 |
| 6 | (SIM)₆ | 3.85 | 5.33 | 4.28 |
| 7 | (SUM)₅ | 4.19 | 7.78 | 4.36 |
| 8 | CNB | 3.65 | 4.33 | 3.87 |
| 9 | DNB | 4.33 | 4.92 | 5.55 |
| 10 | NC | 4.57 | | |

| | | | | |
|---|---|---|---|---|
| *: Each apoptosis ratio is the average of two repeated experiments. | | | | |

Fig. 4A shows that, compared with the negative control, the statistically significant difference of cell apoptosis can only be detected in Group 3, under each concentration condition.

The above results once again indicate that the combination of a multivalent domain and a ligand can achieve effects that is unachievable by either side. The involvement of both the multivalent domain and the ligand promotes phase separation on the cell surface, thereby inducing various cellular events associated with the ligand (such as apoptosis).

In addition, the same recombinant proteins and their combinations were added to the culture medium of SJSA-1 cells by the same method as above, except for the final concentration of each recombinant protein was 200 nM. The Incucyte^{®} Zoom System was used to monitor the survival ratio of each group of cells over time according to the manufacturer's instructions, and the results are shown in Table 2 and Fig. 4B. The time immediately after the addition of recombinant protein or the combination thereof was designated as time "0", and untreated SJSA-1 cells were used as negative control (NC). The cell survival ratio (average proportion of surviving cells) was calculated based on the percentage of non-apoptotic cell area to the total area of the field of view.

**Table 2. Average survival ratio of SJSA-1 cells (%)***

| Time/ h | (SIM)₆-CNB | (SUM)₅ -DNB | (SIM)₆-CNB+ (SUM)₅-DNB | (SIM)₆ | (SUM)₅ | (SIM)₆+ (SUM)₅ | DNB | CNB | DNB+ CNB | NC |
|---|---|---|---|---|---|---|---|---|---|---|
| 0 | 47.27 | 43.30 | 38.37 | 48.78 | 47.45 | 40.37 | 45.08 | 52.39 | 55.49 | 46.01 |
| 0.5 | 48.00 | 44.15 | 37.98 | 48.11 | 46.97 | 41.84 | 46.53 | 53.25 | 54.96 | 46.72 |
| 1 | 49.36 | 45.36 | 35.80 | 49.71 | 48.98 | 43.92 | 47.71 | 54.53 | 56.62 | 47.86 |
| 1.5 | 50.94 | 46.80 | 32.06 | 50.56 | 49.88 | 44.64 | 48.79 | 55.25 | 58.30 | 48.67 |
| 2 | 52.16 | 47.48 | 30.37 | 52.13 | 50.84 | 45.65 | 49.52 | 56.39 | 58.83 | 50.07 |
| 2.5 | 52.92 | 47.79 | 29.39 | 52.63 | 51.51 | 46.03 | 50.08 | 57.26 | 59.61 | 50.69 |
| 3 | 53.54 | 48.20 | 28.98 | 53.87 | 53.05 | 47.07 | 50.74 | 58.55 | 59.85 | 51.76 |
| 3.5 | 54.26 | 49.87 | 28.93 | 55.33 | 53.55 | 47.85 | 51.15 | 59.44 | 61.09 | 52.72 |
| 4 | 55.09 | 50.54 | 29.55 | 55.69 | 54.90 | 48.59 | 52.58 | 61.05 | 62.71 | 53.58 |
| 4.5 | 55.58 | 51.33 | 29.89 | 58.32 | 56.64 | 50.60 | 54.05 | 61.94 | 63.34 | 54.42 |
| 5 | 56.66 | 51.92 | 30.15 | 58.81 | 57.01 | 51.89 | 55.57 | 62.66 | 64.62 | 55.03 |
| 5.5 | 57.46 | 52.37 | 30.61 | 59.01 | 56.63 | 52.11 | 55.43 | 63.56 | 65.53 | 56.26 |
| 6 | 58.83 | 53.69 | 31.21 | 60.05 | 58.35 | 52.86 | 56.80 | 65.25 | 66.90 | 57.26 |
| 6.5 | 60.16 | 54.55 | 31.69 | 60.64 | 60.13 | 53.89 | 57.65 | 66.09 | 67.87 | 58.77 |
| 7 | 60.63 | 55.45 | 32.32 | 61.88 | 60.38 | 55.28 | 58.77 | 66.62 | 68.57 | 59.84 |
| 7.5 | 61.55 | 55.70 | 32.16 | 63.17 | 61.25 | 56.09 | 59.11 | 67.93 | 69.29 | 61.48 |
| 8 | 62.47 | 55.92 | 32.14 | 64.42 | 63.21 | 56.73 | 60.35 | 68.93 | 69.83 | 62.14 |
| 8.5 | 63.09 | 57.22 | 32.80 | 65.21 | 63.44 | 58.06 | 61.41 | 70.04 | 71.31 | 62.80 |
| 9 | 63.92 | 58.37 | 33.11 | 66.75 | 65.18 | 58.47 | 62.32 | 70.22 | 71.96 | 64.82 |
| 9.5 | 65.06 | 58.73 | 33.79 | 67.31 | 66.07 | 59.57 | 63.15 | 71.16 | 72.67 | 65.32 |
| 10 | 65.04 | 59.04 | 34.19 | 68.34 | 65.66 | 60.74 | 63.81 | 71.21 | 72.98 | 66.63 |
| 10.5 | 66.00 | 60.14 | 34.44 | 68.76 | 67.48 | 61.10 | 64.29 | 72.18 | 73.82 | 67.66 |
| 11 | 66.33 | 61.09 | 35.10 | 69.86 | 68.24 | 62.17 | 65.48 | 72.44 | 74.94 | 67.88 |
| 11.5 | 66.97 | 61.33 | 35.59 | 71.30 | 69.11 | 63.37 | 65.66 | 74.12 | 75.06 | 69.63 |

| | | | | | | | | | | |
|---|---|---|---|---|---|---|---|---|---|---|
| *: Each survival ratio is the average of two repeated experiments. | | | | | | | | | | |

The results in Table 2 and Fig. 4B show that, under the same conditions, a decrease in cell viability over time was observed only in the group of adding both recombinant proteins (SUM)₅-DNB and (SIM)₆-CNB; in other words, the group of adding both recombinant proteins (SUM)₅-DNB and (SIM)₆-CNB showed the lowest cell viability at any time point. In contrast, cell viability in the remaining treatment groups increased over time and was not statistically significantly different from that in the negative control.

### Example 4

By the same method as in Example 2, tandemly linked SIM motifs and tandemly linked SUMO3 motifs with lower valency were constructed to obtain multivalent domains (SIM)₃ and (SUM)₃. Among them, (SIM)₃ is obtained by tandemly linking three SIM motifs, and (SUM)₃ is obtained by tandemly linking three SUMO3 motifs.

### 1. Construction of recombinant vectors

**(SIM)₃-CNB expression vector:** SEQ ID NO: 17 was synthesized by DNA fragment synthesis methods known in the art. The vector pRSFDuet-1 was digested with NcoI and XhoI restriction endonucleases, and the aforementioned DNA fragment was ligated into the digested vector by T4 ligase to obtain a recombinant vector, wherein the recombinant vector verified to have correct sequence was designated as pRSFDuet-1-(SIM)₃-CNB. Therefore, pRSFDuet-1-(SIM)₃-CNB can express the fusion protein (SIM)₃-CNB shown in SEQ ID NO: 18 (the fusion protein is also referred to as recombinant protein (SIM)₃-CNB herein).

For ease of understanding, in SEQ ID NO: 17, positions 7-27 encode 7×HisTag, positions 28-48 encode TEV, positions 55-387 encode the multivalent domain (SIM)₃, and positions 388-771 encode the nanobody CNB. The DNA sequence shown in SEQ ID NO: 17 encodes the protein shown in SEQ ID NO: 18. Positions 19-129 in SEQ ID NO: 18 are the amino acid sequence of the multivalent domain (SIM)₃, and positions 130-257 are the amino acid sequence of the nanobody CNB.

**(SUM)₃-DNB expression vector:** by the same method, the DNA fragment shown in SEQ ID NO: 19 was synthesized and ligated into the vector pRSFDuet-1. The obtained recombinant vector with the correct sequence was named pRSFDuet-1-(SUM)₃-DNB. Therefore, pRSFDuet-1-(SUM)₃-DNB can express the fusion protein (SUM)₃-DNB shown in SEQ ID NO: 20 (the fusion protein is also referred to as recombinant protein (SUM)₃-DNB herein).

For ease of understanding, in SEQ ID NO: 19, positions 7-27 encode 7×HisTag, positions 28-48 encode TEV, positions 55-1005 encode a multivalent domain (SUM)₃, and positions 1006-1371 encode a nanobody DNB. The DNA sequence shown in SEQ ID NO: 19 encodes the protein shown in SEQ ID NO: 20. Positions 19-335 in SEQ ID NO: 20 are the amino acid sequence of the multivalent domain (SUM)₃, and positions 336-457 are the amino acid sequence of the nanobody DNB.

**(SIM)₃ Expression vector:** by the same method, the DNA fragment shown in SEQ ID NO: 21 was synthesized and ligated into the vector pRSFDuet-1. The obtained recombinant vector with the correct sequence was named pRSFDuet-1-(SIM)₃. Therefore, pRSFDuet-1-(SIM)₃ can express the recombinant protein (SIM)₃ shown in SEQ ID NO: 22 (also referred to herein as multivalent domain (SIM)₃).

For ease of understanding, in SEQ ID NO: 21, positions 7-27 encode 7×HisTag, positions 28-48 encode TEV, and positions 55-387 encode (SIM)₃. The DNA sequence shown in SEQ ID NO: 21 encodes the protein shown in SEQ ID NO: 22. SEQ ID NO: 22 is the amino acid sequence of multivalent domain (SIM)₃.

**(SUM)₃ expression vector:** by the same method, the DNA fragment shown in SEQ ID NO: 23 was synthesized and ligated into the vector pRSFDuet-1. The obtained recombinant vector with the correct sequence was named pRSFDuet-1-(SUM)₃. Therefore, pRSFDuet-1-(SUM)₃ can express the recombinant protein (SUM)₃ shown in SEQ ID NO: 24 (also referred to herein as multivalent domain (SUM)₃).

For ease of understanding, in SEQ ID NO: 23, positions 7 to 27 encode 7×HisTag, positions 28 to 48 encode TEV, and positions 55 to 1005 encode (SUM)₃. The DNA sequence shown in SEQ ID NO: 23 encodes the protein shown in SEQ ID NO: 24. SEQ ID NO: 24 is the amino acid sequence of multivalent domain (SUM)₃.

### 2. Protein Expression and Purification

The target recombinant protein was expressed using the recombinant vectors pRSFDuet-1-(SIM)₃-CNB, pRSFDuet-1-(SUM)₃-DNB, pRSFDuet-1-(SIM)₃, and pRSFDuet-1-(SUM)₃ obtained in the above "1. Construction of recombinant vectors". By the same purification method as in Example 1, the target recombinant proteins (SIM)₃-CNB, (SUM)₃-DNB, (SIM)₃, and (SUM)₃ were obtained.

### 3. Cell Treatment

By the same method as in Example 2, the total internal reflection fluorescence microscopy (TIRF) imaging of the following recombinant protein combination against the human osteosarcoma cell line SJSA-1 was measured, and the results are shown in Fig. 5:
Treatment group: combination of (SIM)₃-CNB (Red) and (SUM)₃-DNB (Green)
Control group: combination of (SIM)₃ (Red) and (SUM)₃ (Green)

Figs. 5A and 5B show that co-localization of red and green fluorescence was observed in the treatment group, indicating that the added recombinant proteins (SIM)₃-CNB (Red) and (SUM)₃-DNB (Green) aggregated on the cell membrane and formed puncta that are characteristic of liquid-liquid phase separation. Figs. 5A and 5B also show that endogenous receptors DR5 and CXCR4 expressed by cells co-localize with the added recombinant proteins in phase-separated spots. Fig. 5C and Fig. 5D show that phase separation spots formed by the kit of the present invention can be observed on the cell surface, whereas no phase separation spots were observed at all in the control group.

### Example 5

By the same method as in Example 3, the apoptosis-inducing effects of the recombinant proteins (SIM)₃-CNB, (SUM)₃-DNB, (SIM)₃, (SUM)₃ and their combination were determined. The following recombinant proteins and controls were added to the culture medium of SJSA-1 cells to a final concentration of 50 and 100 nM, respectively:
(SIM)₃-CNB (Group 1), (SUM)₃-DNB (Group 2), combination of (SIM)₃-CNB and (SUM)₃-DNB (Group 3), combination of (SIM)₃ and (SUM)₃ (Group 4), combination of DNB and CNB (Group 5), (SIM)₃ (Group 6), (SUM)₃ (Group 7), CNB (Group 8), DNB (Group 9), negative control (Group 10). The results are shown in Table 3 and Fig. 6A.

**Table 3. Average apoptosis ratio of SJSA-1 cells (%)***

| Groups | Recombinant protein or combination thereof | 50 nM | 100 nM |
|---|---|---|---|
| 1 | (SIM)₃-CNB | 3.55 | 3.84 |
| 2 | (SUM)₃-DNB | 5.66 | 8.265 |
| 3 | (SIM)₃-CNB + (SUM)₃-DNB | 47.51 | 44.89 |
| 4 | (SIM)₃ + (SUM)₃ | 4.24 | 4.23 |
| 5 | DNB+CNB | 3.09 | 2.66 |
| 6 | (SIM)₃ | 2.58 | 3.39 |
| 7 | (SUM)₃ | 2.47 | 3.10 |
| 8 | CNB | 3.25 | 2.70 |
| 9 | DNB | 2.96 | 2.65 |
| 10 | NC | 3.05 | |

| | | | |
|---|---|---|---|
| *: The apoptosis ratios of groups 1 to 4 and 10 are the average values of four repeated experiments, and the other groups are the average values of two repeated experiments. | | | |

Fig. 6A shows that, the statistically significant difference compared with the negative control can only be detected in Group 3, under the concentration of both 50 nM and 100 nM.

The above results indicate that the reduction in the number of motifs (i.e., valency) contained in the multivalent domain may have a certain adverse effect on the final effect of inducing cell apoptosis, but the kit of the present invention can still induce phase separation, thereby enhancing the activity or function related to the ligand contained in its components (for example, inducing cell apoptosis in this embodiment). This result also proves that phase separation is the key factor for the recombinant protein combination of the present invention to achieve its manipulation activity.

Furthermore, the survival ratio of SJSA-1 cells over time was measured at a final concentration of 200 nM of each recombinant protein by the same method as in Example 3. The results are shown in Table 4 and Fig. 6B. The time immediately after the addition of the recombinant protein or the combination thereof was designated as time "0", and untreated SJSA-1 cells were used as negative control (NC). The cell survival ratio (average proportion of surviving cells) was calculated based on the percentage of non-apoptotic cell area to the total area of the field of view.

**Table 4. Average survival ratio of SJSA-1 cells (%)***

| Time /h | (SIM)₃-CNB | (SUM)₃-DNB | (SIM)₃-CNB+ (SUM)₃-DNB | (SIM)₃ | (SUM)₃ | (SIM)₃+ (SUM)₃ | DNB | CNB | DNB+ CNB | NC |
|---|---|---|---|---|---|---|---|---|---|---|
| 0 | 41.73 | 41.38 | 47.01 | 53.61 | 56.57 | 45.40 | 45.08 | 52.39 | 55.49 | 46.01 |
| 0.5 | 41.55 | 41.18 | 42.39 | 53.64 | 56.78 | 46.91 | 46.53 | 53.25 | 54.96 | 46.72 |
| 1 | 42.81 | 42.29 | 34.62 | 55.67 | 57.63 | 47.39 | 47.71 | 54.53 | 56.62 | 47.86 |
| 1.5 | 44.63 | 44.33 | 31.63 | 57.28 | 59.83 | 48.31 | 48.79 | 55.25 | 58.30 | 48.67 |
| 2 | 45.71 | 45.92 | 31.31 | 58.24 | 61.36 | 49.58 | 49.52 | 56.39 | 58.83 | 50.07 |
| 2.5 | 46.68 | 47.05 | 31.48 | 59.47 | 61.89 | 50.72 | 50.08 | 57.26 | 59.61 | 50.69 |
| 3 | 46.99 | 48.47 | 32.05 | 60.46 | 62.75 | 51.53 | 50.74 | 58.55 | 59.85 | 51.76 |
| 3.5 | 47.78 | 48.82 | 32.93 | 61.95 | 63.90 | 52.94 | 51.15 | 59.44 | 61.09 | 52.72 |
| 4 | 49.12 | 49.18 | 33.74 | 63.22 | 64.72 | 53.83 | 52.58 | 61.05 | 62.71 | 53.58 |
| 4.5 | 49.40 | 50.49 | 34.17 | 63.12 | 65.37 | 55.63 | 54.05 | 61.94 | 63.34 | 54.42 |
| 5 | 50.35 | 50.68 | 34.55 | 64.64 | 66.51 | 56.34 | 55.57 | 62.66 | 64.62 | 55.03 |
| 5.5 | 51.21 | 51.15 | 35.38 | 64.10 | 68.06 | 57.42 | 55.43 | 63.56 | 65.53 | 56.26 |
| 6 | 52.81 | 53.66 | 35.64 | 65.61 | 69.14 | 57.51 | 56.80 | 65.25 | 66.90 | 57.26 |
| 6.5 | 52.62 | 54.70 | 36.22 | 67.13 | 70.02 | 58.79 | 57.65 | 66.09 | 67.87 | 58.77 |
| 7 | 53.78 | 56.07 | 36.96 | 68.15 | 71.23 | 59.42 | 58.77 | 66.62 | 68.57 | 59.84 |
| 7.5 | 54.77 | 57.38 | 37.40 | 69.25 | 71.88 | 60.55 | 59.11 | 67.93 | 69.29 | 61.48 |
| 8 | 56.06 | 58.30 | 37.52 | 70.05 | 72.72 | 61.53 | 60.35 | 68.93 | 69.83 | 62.14 |
| 8.5 | 56.63 | 59.71 | 37.94 | 70.48 | 74.16 | 63.12 | 61.41 | 70.04 | 71.31 | 62.80 |
| 9 | 57.43 | 60.06 | 38.33 | 70.72 | 75.16 | 63.96 | 62.32 | 70.22 | 71.96 | 64.82 |
| 9.5 | 57.97 | 61.51 | 38.92 | 72.16 | 75.94 | 64.64 | 63.15 | 71.16 | 72.67 | 65.32 |
| 10 | 59.25 | 62.52 | 39.60 | 72.77 | 76.29 | 65.66 | 63.81 | 71.21 | 72.98 | 66.63 |
| 10.5 | 59.81 | 63.63 | 40.26 | 73.68 | 77.41 | 66.31 | 64.29 | 72.18 | 73.82 | 67.66 |
| 11 | 60.29 | 65.06 | 40.21 | 74.86 | 78.40 | 67.56 | 65.48 | 72.44 | 74.94 | 67.88 |
| 11.5 | 61.37 | 67.07 | 41.14 | 76.16 | 79.04 | 68.51 | 65.66 | 74.12 | 75.06 | 69.63 |

| | | | | | | | | | | |
|---|---|---|---|---|---|---|---|---|---|---|
| *: Each survival ratio is the average of two repeated experiments. | | | | | | | | | | |

The results in Table 4 and Fig. 6B show that, under the same conditions, a decrease in cell viability over time was observed in the treatment group to which a combination of the recombinant proteins (SUM)₃-DNB and (SIM)₃-CNB was added. In contrast, the cell survival ratios of the other treatment groups increased over time and were not statistically significantly different from those of the negative control.

### Example 6

Activation of caspase 3 was mediated by cleavage of full-length sequence, which is the key event in the process of apoptosis (see, for example, Nicholson, Donald W. et al., "Identification and inhibition of the ICE/CED-3 protease necessary for mammalian apoptosis." Nature 376.6535 (1995): 37-43; Hsia, Jiun-Yi et al., "Prognostic significance of caspase-3 expression in primary resected esophageal squamous cell carcinoma." European Journal of Surgical Oncology (EJSO) 29.1 (2003): 44-48). PARP, as a substrate of activated caspase 3, will produce an 85Kd cleavage product after cleavage. The cleavage of PARP is also considered to be a sign of apoptosis (Lazebnik et al., Nature 371(6495): 346-347, 1994). The induction effect of the recombinant protein of the present invention on cell apoptosis was verified by analyzing the expression level of pro-caspase-3 and the cleavage products of PARP in SJSA-1 cells.

Specifically, recombinant proteins (SIM)₃, (SUM)₃, (SIM)₃-CNB, (SUM)₃-DNB, CNB and DNB were added to the culture medium of SJSA-1 cells (1×10⁶ cells/well) in the following combinations to a final concentration of 50 nM for each recombinant protein:
Combination of (SIM)₃-CNB and (SUM)₃-DNB (Group 1), (SUM)-DNB (Group 2), (SIM)₃-CNB (Group 3), combination of DNB and CNB (Group 4), combination of (SIM)₃ and (SUM)₃ (Group 5).

After adding the recombinant protein, the cells were incubated at 37°C for 4 hr. Then, SJSA-1 cell lysate was prepared using Minute^{™} Total Protein Extraction Kit for Animal Cultured Cells/Tissues (purchased from Invent Biotech) according to the manufacturer's instructions. The levels of pro-caspase-3 and PARP cleavage products in SJSA-1 cell lysates were determined using Western blotting methods commonly known in the art (see, for example, Zhang, T. et al., "Discovery of a novel third-generation EGFR inhibitor and identification of a potential combination strategy to overcome resistance." Molecular Cancer 19.1 (2020)). The assay used β-tubulin as an internal control, rabbit anti-Caspase-3 polyclonal antibody (purchased from CST, Catalog No. 9662S), rabbit anti-PARP antibody (purchased from CST, Catalog No. 9542s), and rabbit anti-Tublin antibody (purchased from Gene Protein Link, Catalog No. p01106) as primary antibodies, and F(ab')₂-goat anti-rabbit IgG (H+L), HRP as secondary antibody. The results are shown in Fig. 6C.

The results showed that a decrease in the amount of procaspase-3 and an 85 kDa band generated by cleavage of the PARP protein were detected in Group 1. This indicates that, when the recombinant proteins (SIM)₃-CNB and (SUM)₃-DNB are added simultaneously, the cells undergo characteristic intracellular events of apoptosis. In contrast, addition of the multivalent domain, specific ligand molecule, or their combination alone failed to induce apoptosis.

### Example 7

The present inventors further verified the phase transition adjusting ability and apoptosis inducing activity of the recombinant protein of the present invention in other cell lines.

The cell lines to be tested were seeded in a 24-well plate (Falcon) at a density of 1×10⁶ cells/well, and the following recombinant proteins or their combinations were added respectively, and reacted at 37°C, 5% CO₂ for 4 hr. By the same method as in Example 3, flow cytometry analysis was performed. Specifically, the cells were washed with PBS buffer, and then the fluorescence intensity was measured using a flow cytometer LSRFortessa SORP (BD), and flow cytometry based on the Annexin V-FITC/PI double staining method (Beyotime) was performed to determine the apoptotic ratio of the cells.
Treatment Group 1: (SIM)₃-CNB
Treatment Group 2: (SUM)₃-DNB
Treatment Group 3: Combination of (SIM)₃-CNB and (SUM)₃-DNB
Treatment group 4: combination of (SIM)₃ and (SUM)₃
Treatment Group 5: Combination of CNB and DNB
Treatment group 6: negative control NC.

### 1. Apoptosis-inducing activity against the cell line COLO205

The cell line COLO-205 was derived from human colon adenocarcinoma and was purchased from ATCC. It was maintained in RMPI 1640 containing 10% fetal bovine serum and 100 Units/ml Pen-Strep at 37°C and 5% CO₂. The above recombinant proteins or their combination were added to the cell culture medium at a final concentration of 10, 20, or 50 nM, respectively, and the cell apoptosis ratio was determined by flow cytometry. The results are shown in Table 5 and Fig. 7A.

**Table 5: Average apoptosis ratio (%) of human colorectal cancer cell line COLO205***

| Groups | Recombinant protein or | 10 nM | 20 nM | 50 nM |
|---|---|---|---|---|
| | combination thereof | | | |
| 1 | (SIM)₃-CNB | 11.27 | 9.71 | 9.80 |
| 2 | (SUM)₃-DNB | 10.51 | 9.16 | 10.48 |
| 3 | (SIM)₃-CNB + (SUM)₃-DNB | 58.86 | 77.50 | 91.25 |
| 4 | (SIM)₃ + (SUM)₃ | 10.29 | 10.96 | 10.38 |
| 5 | DNB+CNB | 12.13 | 12.79 | 12.53 |
| 6 | NC | 11.97 | | |

| | | | | |
|---|---|---|---|---|
| *: Groups 4 and 5 are the arithmetic means of two repeated experiments, and the rest of the groups are the arithmetic means of four repeated experiments. | | | | |

### 2. Apoptosis-inducing activity against the Jurkat cell line

The cell line Jurkat was derived from human T lymphocytic leukemia and was purchased from the China National Biomedical Experimental Cell Resource Bank (http://www.crcpumc.com/) and maintained in RMPI 1640 containing 10% fetal bovine serum and 100 Units/ml Pen-Strep at 37°C and 5% CO₂. The above recombinant proteins or their combination were added to the cell culture medium at a final concentration of 10, 20, or 50 nM, respectively, and the cell apoptosis ratio was determined by flow cytometry. The results are shown in Table 6 and Fig. 7B.

**Table 6: Average apoptosis ratio of human T lymphocyte leukemia cell line Jurkat (%)***

| Groups | Recombinant protein or combination thereof | 10 nM | 20 nM | 50 nM |
|---|---|---|---|---|
| 1 | (SIM)₃-CNB | 4.69 | 4.50 | 5.15 |
| 2 | (SUM)₃-DNB | 4.22 | 4.10 | 4.29 |
| 3 | (SIM)₃-CNB + (SUM)₃-DNB | 51.71 | 65.91 | 61.72 |
| 4 | (SIM)₃ + (SUM)₃ | 4.55 | 4.75 | 4.30 |
| 5 | DNB+CNB | 4.50 | 5.10 | 3.55 |
| 6 | NC | 4.51 | | |

| | | | | |
|---|---|---|---|---|
| *: Groups 4 and 5 are the arithmetic means of two repeated experiments, and the rest of the groups are the arithmetic means of four repeated experiments. | | | | |

### 3. Apoptosis-inducing activity against the cell line OCI-AML3

The cell line OCI-AML3 was derived from human acute myeloid leukemia and was purchased from the China National Biomedical Experimental Cell Resource Bank (http://www.crcpumc.com/) and maintained in RMPI-1640 containing 20% fetal bovine serum and 100 Units/ml Pen-Strep at 37°C and 5% CO₂. The above recombinant proteins or their combination were added to the cell culture medium at a final concentration of 20, 50, or 100 nM, respectively, and the cell apoptosis ratio was determined by flow cytometry. The results are shown in Table 7 and Fig. 7C.

**Table 7: Average apoptosis ratio (%) of human acute myeloid leukemia OCI-AML3***

| Groups | Recombinant protein or combination thereof | 20 nM | 50 nM | 100 nM |
|---|---|---|---|---|
| 1 | (SIM)₃-CNB | 4.86 | 5.16 | 4.86 |
| 2 | (SUM)₃-DNB | 4.49 | 4.50 | 4.68 |
| 3 | (SIM)₃-CNB + (SUM)₃-DNB | 29.03 | 40.69 | 41.89 |
| 4 | (SIM)₃ + (SUM)₃ | / | / | 7.43 |
| 5 | DNB+CNB | / | / | 5.17 |
| 6 | NC | 4.00 | | |

| | | | | |
|---|---|---|---|---|
| *: Each group is the arithmetic mean of four repeated experiments, and "/" means no measurement was performed. | | | | |

### 4. Apoptosis-inducing activity against the cell line MIA PaCa-2

The cell line MIA PaCa-2 was derived from human pancreatic cancer and was purchased from the China National Biomedical Experimental Cell Resource Bank (http://www.crcpumc.com/) and maintained in RMPI-1640 containing 20% fetal bovine serum and 100 Units/ml Pen-Strep at 37°C and 5% CO₂. The above recombinant proteins or their combination were added to the cell culture medium at a final concentration of 20, 50, or 100 nM, respectively, and the cell apoptosis ratio was determined by flow cytometry. The results are shown in Table 8 and Fig. 7D.

**Table 8: Average apoptosis ratio (%) of human pancreatic cancer cell line MIA PaCa-2***

| Groups | Recombinant protein or combination thereof | 20 nM | 50 nM | 100 nM |
|---|---|---|---|---|
| 1 | (SIM)₃-CNB | 8.45 | 8.48 | 11.08 |
| 2 | (SUM)₃-DNB | 8.70 | 8.10 | 8.45 |
| 3 | (SIM)₃-CNB + (SUM)₃-DNB | 26.78 | 25.63 | 25.65 |
| 4 | (SIM)₃ + (SUM)₃ | 13.53 | 16.28 | 15.23 |
| 5 | DNB+CNB | 7.43 | 8.43 | 9.68 |
| 6 | NC | 7.05 | | |

| | | | | |
|---|---|---|---|---|
| *: Each group is the arithmetic mean of four repeated experiments. | | | | |

### 5. Apoptosis-inducing activity against HEK293 cell line

The cell line HEK293 was derived from human embryonic kidney and purchased from the China National Biomedical Experimental Cell Resource Bank (http://www.crcpumc.com/) and maintained in DMEM containing 10% fetal bovine serum and 100 Units/ml Pen-Strep at 37°C and 5% CO₂. The above recombinant proteins or their combination were added to the cell culture medium at a final concentration of 20, 50, or 100 nM, respectively, and the cell apoptosis ratio was determined by flow cytometry. The results are shown in Table 9 and Fig. 7E.

**Table 9: Average apoptosis ratio of human embryonic kidney cell line HEK293 (%)***

| Groups | Recombinant protein or combination thereof | 20 nM | 50 nM | 100 nM |
|---|---|---|---|---|
| 1 | (SIM)₃-CNB | 3.68 | 2.42 | 2.30 |
| 2 | (SUM)₃-DNB | 2.32 | 3.14 | 2.90 |
| 3 | (SIM)₃-CNB + (SUM)₃-DNB | 3.69 | 2.69 | 4.28 |
| 4 | (SIM)₃ + (SUM)₃ | 4.99 | 5.31 | 3.09 |
| 5 | DNB+CNB | 6.23 | 6.20 | 7.93 |
| 6 | NC | 2.64 | | |

| | | | | |
|---|---|---|---|---|
| *: The 20 nM and 50 nM treatments in groups 1 to 4, and the 20 nM, 50 nM and 100 nM treatments in group 5 are the arithmetic means of two repeated experiments, and the concentrations in the other groups are the arithmetic means of four repeated experiments. | | | | |

The results in Tables 5 to 8 and Figs. 7A to 7D show that in the cell lines COLO205, Jurkat, OCI-AML3 and MIA PaCa-2, the combination of recombinant proteins (SUM)₃-DNB and (SIM)₃-CNB can induce significant cell apoptosis at all concentrations compared with the negative control, showing a significant synergistic effect compared with the use of recombinant protein (SUM)₃-DNB or (SIM)₃-CNB alone. In addition, apoptosis weren't caused in all these cell lines by either using the component of the above combinations (i.e., recombinant proteins (SUM)₃-DNB or (SIM)₃-CNB alone), or using the combination of multivalent domains (SUM)₃ and (SIM)₃, or using the combination of nanobodies DNB and CNB.

On the other hand, the results in Table 9 and Fig. 7E show that in the cell line HEK293T used as a negative control, neither the recombinant protein nor the combination thereof was able to induce significant cell apoptosis compared with the negative control at any concentration.

### Example 8

This example measured the expression levels of CXC motif chemokine receptor 4 (CXCR4) and death receptor 5 (DR5) in cell lines COLO205, Jurkat, OCI-AML3, SJSA-1, MIA PaCa-2 and HEK293 by Western blotting commonly known in the art, so as to further confirm that the fusion protein of the present invention promotes phase separation on the basis of its binding to cell surface receptors, and manipulates cell apoptosis,. The assay used β-tubulin as an internal control, rabbit anti-DR5 monoclonal antibody (purchased from Abcam, catalog number ab8416), rabbit anti-CXCR4 antibody (purchased from Protein Tech, catalog number 60042-1-Ig), and rabbit anti-Tublin antibody (purchased from Gene Protein Link, catalog number p01106) as primary antibodies, and F(ab')2-goat anti-rabbit IgG (H+L), HRP as a secondary antibody (Thermo Fisher Scientific, catalog number A24537). The results are shown in Fig. 8.

The results showed that the expression of CXCR4 receptor could be detected in all the aforementioned cell lines, after correction by internal control protein. However, the expression of DR5 receptor was detected only in the cell lines COLO205, Jurkat, OCI-AML3, MIA PaCa-2 and SJSA-1, which were just the cell lines responded to the recombinant proteins (SUM)₃-DNB and (SIM)₃-CNB of the present invention and became apoptosis. In contrast, in the cell line HEK293 that failed to respond to the phase transition adjusting fusion protein of the present invention, the expression of the death receptor DR5 was not detected as expected.

The above results indicate that the combination of phase transition adjusting fusion proteins of the present invention specifically acts on cell surface receptor molecules through the ligands to which they are fused, thereby promoting receptor molecule aggregation through phase separation, affecting its downstream signaling pathway, and inducing cell apoptosis.

### Example 9

Using a method similar to the above example, the multivalent domain was replaced with other multivalent domains that have been shown to undergo phase separation, namely tandemly repeated PRMH and tandemly repeated SH3, to study the relationship between the multivalent domain and phase separation and cell apoptosis.

Five PRMH motifs and five SH3 motifs were tandemly linked to form (PRMH)₅ and (SH3)₅, which were fused with CNB and DNB to generate fusion proteins (PRMH)₅-CNB and (SH3)₅-DNB.

### 1. Construction of recombinant vectors

**(PRMH)₅-CNB expression vector:** the DNA fragment shown in SEQ ID NO: 25 was synthesized by a DNA fragment synthesis method known in the art. PCR amplification was performed using primer pair pRSFDuet-1-(PRMH)₅-CNB-F and pRSFDuet-1-(PRMH)₅-CNB-R and the DNA fragment shown in SEQ ID NO: 25 as a template to obtain the recombinant vector pRSFDuet-1-(PRMH)₅-CNB.
pRSFDuet-1-(PRMH)₅-CNB-F:
   ACTTTAATAAGGAGATATACCATGAAACATCATCATCATCA (SEQ ID NO:45)
pRSFDuet-1-(PRMH)₅-CNB-R:
   TTCTTTACCAGACTCGAGttaAGAGCTTACGGTAACCTG (SEQ ID NO: 46).

pRSFDuet-1-(PRMH)₅-CNB can express the fusion protein shown in SEQ ID NO: 26, hereinafter also referred to as recombinant protein (PRMH)₅-CNB.

For ease of understanding, in SEQ ID NO: 25, positions 7-27 encode 7×HisTag, positions 28-48 encode TEV, positions 79-504 encode (PRMH)₅, and positions 505-888 encode CNB. The DNA sequence shown in SEQ ID NO: 25 encodes the protein shown in SEQ ID NO: 26. In SEQ ID NO: 26, positions 27-168 are the amino acid sequence of (PRMH)₅, and positions 169-296 are the amino acid sequence of CNB. Positions 27 to 40 of sequence 26 are an amino acid sequence of a PRMH motif.

**(SH3)₅-DNB expression vector:** the DNA fragment shown in SEQ ID NO: 27 was synthesized in the same manner. The primer pair pRSFDuet-1-(SH3)₅-DNB-F and pRSFDuet-1-(SH3)₅-DNB-R were used to perform PCR amplification with the obtained DNA fragment as a template to obtain the recombinant vector pRSFDuet-1-(SH3)₅-DNB.
pRSFDuet-1-(SH3)₅-DNB-F:
   ACTTTAATAAGGAGATATACCATGAAACATCATCATCATCAT (SEQ ID NO: 47)
pRSFDuet-1-(SH3)₅-DNB-R:
   TTCTTTACCAGACTCGAGttaAGAGCTTACGGTAACGAG (SEQ ID NO: 48).

pRSFDuet-1-(SH3)₅-DNB can express the fusion protein shown in SEQ ID NO: 28, which is also referred to as recombinant protein (SH3)₅-DNB hereinafter.

For ease of understanding, in SEQ ID NO: 27, positions 7-27 encode 7×HisTag, positions 28-48 encode TEV, positions 55-1284 encode (SH3)₅, and positions 1285-1650 encode DNB. The DNA molecule shown in SEQ ID NO: 27 encodes the protein shown in SEQ ID NO: 28. In SEQ ID NO: 28, positions 19 to 428 are the amino acid sequence of (SH3)₅, and positions 429 to 550 are the amino acid sequence of DNB. Positions 19 to 81 of SEQ ID NO: 28 are an amino acid sequence of an SH3 motif.

### 2. Protein Expression and Purification

The target recombinant protein was expressed using the recombinant vectors pRSFDuet-1-(PRMH)₅-CNB and pRSFDuet-1-(SH3)₅-DNB obtained in the above "1. Construction of recombinant vector". By the same purification method as in Example 1, the target recombinant proteins (PRMH)₅-CNB and (SH3)₅-DNB were obtained.

### 3. Cell Treatment

According to the manufacturer's instructions, the recombinant protein (SH3)₅-DNB obtained in the above "2. Protein expression and purification" was labeled with Alexa647 (ThermoFisher, A20006)-Green. The obtained recombinant protein labeled with Alexa647-Green was recorded as (SH3)₅-DNB (Green). Also according to the manufacturer's instructions, the recombinant protein (PRMH)₅-CNB obtained in the above "2. Protein expression and purification" was labeled with Alexa594 (ThermoFisher, A20004)-Red, and the obtained recombinant protein labeled with Alexa594-Red was recorded as (PRMH)₅-CNB (Red).

The recombinant proteins (PRMH)₅-CNB (Red) and (SH3)₅-DNB (Green) were added to the culture medium of SJSA-1 cells (RMPI-1640 culture medium containing 10% FBS and 1% penicillin-streptomycin) to a final concentration of 100 nM, mixed and incubated for 1 minute, and then laser confocal scanning microscopy imaging was performed in the same manner as in Example 1. The results are shown in Fig. 9A.

The results showed that after the addition of the combination of recombinant proteins, puncta characteristic of phase separation were observed on the cell membrane of the SJSA-1 cell line, and the green fluorescent signal (which represents (PRMH)₅-CNB) and the red fluorescent signal (which represents (SH3)₅-DNB) were co-localized in the puncta.

This result is consistent with the result observed in Example 1, in which a combination of (SIM)₆-R4L (Red) and (SUM)₅-TRAIL (Green) was used, indicating that changing the type of fused multivalent domains did not affect the fusion protein-mediated phase separation.

The recombinant proteins were added to the culture medium of SJSA-1 cells in the following manner to a final concentration of 100 nM, mixed and incubated for 4 hours, and then the apoptotic ratio of cells in each group was determined by flow cytometry as in Example 3. The results are shown in Fig. 9B.
Treatment group 1: negative control NC
Treatment group 2: (PRMH)₅-CNB (Red)
Treatment group 3: (SH3)₅-DNB (Green)
Treatment group 4: combination of (PRMH)₅-CNB (Red) and (SH3)₅-DNB (Green).

Fig. 9B shows that the apoptotic ratios of treatment group 1(negative control), treatment group 2 ( the addition of recombinant protein (PRMH)₅-CNB (Red) alone), and treatment group 3 (the addition of (SH3)₅-DNB (Green) alone) were approximately the same (6.11%, 6.26%, and 6.44%, respectively). In contrast, in treatment group 4, where (PRMH)₅-CNB (Red) and (SH3)₅-DNB (Green) were added simultaneously, approximately 58.4% of the cells underwent apoptosis, and the apoptotic ratio was significantly increased. This once again indicates that the recombinant protein combination of the present invention can induce cell apoptosis and that this effect is not dependent on a specific multivalent domain.

The experiments in Examples 1 to 9 show that the recombinant fusion protein obtained by linking the multivalent domain to a ligand (such as an antibody, cytokine) that can target specific cell surface molecules (such as tumor antigens or membrane receptors) can form a synergistic kit. Under conditions suitable for phase separation, the components of the kit can regulate the aggregation or oligomerization of the target protein through intermolecular interactions, thereby achieving the manipulation of various cellular events related to the target protein, such as enhancing or inhibiting cell apoptosis. Theoretically, the kit of the present invention can be used as a new tool to regulate all intracellular and extracellular events related to cell surface molecules, and has broad application prospects in the fields of disease diagnosis, treatment, molecular detection, drug screening, etc. Furthermore, those skilled in the art will appreciate that the above technical effects of the kit of the present invention are not limited to the scope of the specific multivalent domains or specific ligands used in the examples of this specification.

The present invention has been described in detail above. It is obvious to those skilled in the art that the present invention can be implemented in a wider range under equivalent parameters, concentrations and conditions without departing from the spirit and scope of the present invention and without unnecessary experiments. While the present invention has been described in detail with reference to specific embodiments, it will be appreciated that the invention is capable of further modifications. In short, according to the principles of the present invention, this application is intended to include any changes, uses or improvements to the present invention, including changes that depart from the scope disclosed in this application and are made using conventional techniques known in the art. Some essential features may be applied within the scope of the following claims.

## Claims

1. A phase transition adjusting kit, **characterized in that** the kit comprises multiple components including a first component and a second component, wherein the first component comprises a first multivalent domain and a first ligand that specifically binds to a first cell surface molecule, and the second component comprises a second multivalent domain and a second ligand that specifically binds to a second cell surface molecule, the first cell surface molecule is the same as or different from the second cell surface molecule, and, each of the components comprises no more than one ligand, and
wherein the kit regulates phase transition of the kit itself or the cell surface molecules bound to the kit through interactions between the multivalent domains of the multiple components contained therein.

2. The phase transition adjusting kit of claim 1, comprising three or more components, wherein the components other than the first component and the second component comprise a domain capable of regulating phase transition via the interaction with the first and/or second multivalent domains, and optionally contain or do not contain a third ligand that specifically binds to a third cell surface molecule.

3. The phase transition adjusting kit of any one of the preceding claims, wherein the ligands contained in the components are covalently linked to the multivalent domains, optionally via a linker, which the linker may be a peptide linker or a non-peptide linker.

4. The phase transition adjusting kit of any one of the preceding claims, wherein the multivalent domain in the component comprises at least two tandemly linked motifs, for example, the number of the tandemly linked motifs is 3, 4, 5, 6, 7, 8, 9 or 10.

5. The phase transition adjusting kit of any one of the preceding claims, wherein the multivalent domain comprises at least two tandemly linked SUMO3 motifs having the sequence shown in SEQ ID NO: 49 or the sequencehaving at least 80%, 90%, 95%, 99% sequence identity to the SUMO3 motif shown in SEQ ID NO: 49, or at least two tandemly linked SIM motifs having the sequence shown in SEQ ID NO: 50 or the sequence having at least 80%, 90%, 95%, 99% sequence identity to the SIM motif shown in SEQ ID NO: 50, or at least two tandemly linked PRMH motifs having the sequence shown in SEQ ID NO: 51 or the sequence having at least 80%, 90%, 95%, 99% sequence identity to the PRMH motif shown in SEQ ID NO: 51, or at least two tandemly linked SH3 motifs having the sequence shown in SEQ ID NO: 52 or the sequence having at least 80%, 90%, 95%, 99% sequence identity to the SH3 motif shown in SEQ ID NO: 52.

6. The phase transition adjusting kit of any one of the preceding claims, wherein the first cell surface molecule is a tumor necrosis factor receptor, preferably a death receptor 5 (DR5) or a Fas receptor, more preferably a death receptor 5 (DR5).

7. The phase transition adjusting kit of any one of the preceding claims, wherein the second cell surface molecule is a tumor-associated antigen, preferably the tumor-associated antigen is selected from the group consisting of CXC motif chemokine receptor 4 (CXCR4), hepatocyte growth factor receptor (c-Met or HGFR), epidermal growth factor receptor (EGFR), human epidermal growth factor receptor 2 (HER2) and prostate specific membrane antigen (PSMA), most preferably CXC motif chemokine receptor 4 (CXCR4), fibroblast activation protein (FAP), carcinoembryonic antigen (CEA), folate receptor alpha (FolR1), melanoma-associated chondroitin sulfate proteoglycan (MCSP), p95HER2, EpCAM, HER3, CD30 or TPBG (5T4), CD19, CD79b, CD20, CD22, CD37, CD38, BCMA and GPRC5D.

8. The phase transition adjusting kit of any one of the preceding claims, wherein the ligand may be selected from a peptide ligand or a non-peptide ligand, and the peptide ligand is preferably selected from one or more of the group consisting of: an antibody or an antigen-binding fragment thereof, a cytokine, a growth factor, an adhesion molecule, a peptide hormone, or a polypeptide randomly selected by phage display, yeast display or the like that specifically binds to a cell surface molecule; the antibody is more preferably selected from a monoclonal antibody, a polyclonal antibody, a human antibody or a humanized antibody; the antigen-binding fragment may be selected from F(ab')₂, Fab, a single-chain variable fragment (scFv), a single-domain antibody fragment (VHH or nanobody); and the non-peptide ligand is preferably selected from one or more of the group consisting of a small molecule agonist or antagonist, an antisense oligonucleotide or a small interfering RNA (siRNA).

9. The phase transition adjusting kit of any one of the preceding claims, wherein the first ligand and the second ligand are both single-chain variable fragments, preferably, the first ligand in the form of a single-chain variable fragment comprises the amino acid sequence shown in SEQ ID NO: 53 or the amino acid sequence having at least 80%, 90%, 95%, 99% identity to the amino acid sequence shown in SEQ ID NO: 53, and the second ligand in the form of a single-chain variable fragment comprises the amino acid sequence shown in SEQ ID NO: 54 or the amino acid sequence having at least 80%, 90%, 95%, 99% identity to the amino acid sequence shown in SEQ ID NO: 54.

10. The phase transition adjusting kit of any one of the preceding claims,
wherein the first component comprises the amino acid sequence of positions 27 to 673 of SEQ ID NO: 12, or the amino acid sequence of positions 19 to 457 of SEQ ID NO: 20, or the amino acid sequence of positions 19 to 550 of SEQ ID NO: 28, or comprises an amino acid sequence having at least 80%, 90%, 95%, 99% identity to the sequence selected from positions 27 to 673 of SEQ ID NO: 12, positions 19 to 457 of SEQ ID NO: 20, or positions 19 to 550 of SEQ ID NO: 28; and
the second component comprises the amino acid sequence of positions 21 to 373 of SEQ ID NO: 10, or the amino acid sequence of positions 19 to 257 of SEQ ID NO: 18, or the amino acid sequence of positions 27 to 296 of SEQ ID NO: 26, or comprises an amino acid sequence having at least 80%, 90%, 95%, 99% identity to the sequence selected from positions 21 to 373 of SEQ ID NO: 10, positions 19 to 257 of SEQ ID NO: 18, or positions 27 to 296 of SEQ ID NO: 26.

11. The phase transition adjusting kit of any one of the preceding claims, further comprising a tag that does not affect the functions of the ligand and the multivalent domain and/or a polypeptide that cleaves the tag.

12. A method for screening phase transition adjusting kit, the method comprising the following steps:
Step A: generating a library comprising a plurality of candidate phase transition adjusting components, wherein each of the candidate phase transition adjusting components comprises a multivalent domain and comprises or dose not comprise no more than one ligand capable of specifically binding to a cell surface molecule;
Step B: arbitrarily selecting at least two of the candidate phase transition adjusting components from the library generated in step A, and combining them into a candidate phase transition adjusting kit, wherein the cell surface molecules recognized by the candidate components in the candidate kit via the ligands are the same as or different from each other; and
Step C: under conditions suitable for phase transition, measuring the activity of the candidate phase transition adjusting kit combined in step B in generating phase transition droplets, and selecting the candidate phase transition adjusting kit that generates more phase transition droplets than the negative control as the target kit, wherein the negative control is a parallel measurement not containing the candidate phase transition adjusting kit.

13. A pharmaceutical composition comprising the phase transition adjusting kit according to any one of the preceding claims, and optionally a pharmaceutically acceptable carrier,
preferably, the pharmaceutical composition is a reagent kit.

14. Use of the phase transition adjusting kit or pharmaceutical composition of any one of the preceding claims in the preparation of a medicament for treating a disease, wherein the disease can be selected from the group consisting of chronic autoimmune disorders, inflammatory disorders, diseases associated with abnormal cell proliferation or abnormal cell apoptosis, sepsis or viral infection, preferably the disease associated with abnormal cell proliferation or abnormal cell apoptosis is a cancer, more preferably the cancer is selected from lung cancer, colorectal cancer, head and neck cancer (e.g., head and neck squamous cell carcinoma), glioma (e.g., glioma), neuroblastoma, melanoma, breast cancer, bladder cancer, kidney cancer, ovarian cancer, pancreatic cancer, cervical cancer, esophageal cancer, sarcoma, esophageal cancer (e.g., esophageal squamous cell carcinoma), thyroid cancer (e.g., papillary thyroid cancer) or prostate cancer, B-cell lymphoma, chronic lymphocytic leukemia (CLL), chronic myeloid leukemia, diffuse large B-cell lymphoma (DLBCL), follicular lymphoma, myelodysplastic syndrome (MDS), non-Hodgkin lymphoma (NHL), acute lymphocytic leukemia (ALL), acute monocytic leukemia, multiple myeloma, acute myeloid leukemia (AML), mixed lineage leukemia, NUT midline carcinoma, Burkitt's lymphoma or mycosis fungoides (MF), or metastatic forms thereof.

15. A product comprising (a) a phase transition adjusting kit of any one of claims 1 to 11, and (b) a receptor internalization inhibitor, for simultaneous, separate or sequential use as a combined preparation in the treatment of a disease.
